# EUROPEAN PATENT APPLICATION

(11) **EP 2 100 880 A1**
(43) Date of publication of application: **16.09.2009**
(21) Application number: 07831976.1
(22) Date of filing: 09.11.2007
(51) Int. Cl.: C07D 209/86, C07D 209/88, C09K 11/06, H01L 51/50

(54) **NOVEL CARBAZOLE DERIVATIVE AND USE THEREOF**

(30) Priority: 16.11.2006 JP 2006310825
(71) Applicant: Bando Chemical Industries, Ltd., Kobe-shi, Hyogo 652-0883 (JP)
(72) Inventor: YOKOTA, Masami, Osaka 5550033 (JP); TSUBAKI, Tomoyuki, Kobe-shi Hyogo 6520883 (JP)
(74) Representative: Benson, John Everett
(86) International application number: PCT/JP2007/072246
(87) International publication number: WO 2008/059943

(57) **Abstract**

The invention discloses a novel carbazole derivative which has at least two carbazole structures in the molecule, can form a stable amorphous film by itself at normal temperature or higher, has a high glass transition temperature, and can be suitably used as an organo-electronic functional material. The following compound having the formula 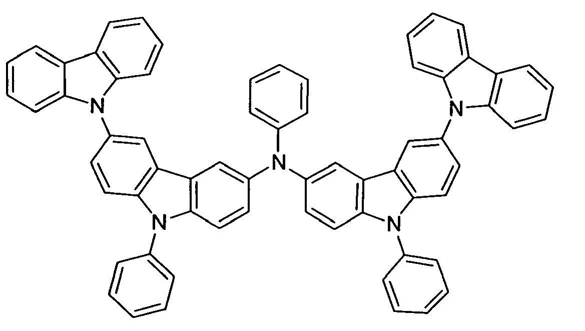 is mentioned as a representative example.

## Description

### Technical Field

This invention relates to novel carbazole derivatives and use thereof. More particularly, the invention relates to novel carbazole derivatives which form amorphous film at normal temperature or higher, but also have a high glass transition temperature, and hence excellent heat resistance, and moreover, which hardly crystallize if they are heated. The invention further relates to use of such carbazole derivatives as an organo-electronic functional material, in particular, as a hole injecting and/or transporting agent, and to an organic electroluminescence element having a hole injecting and/or transporting layer comprising such a hole injecting and/or transporting agent.

### Background

Among organic materials having a photoelectric function which produces electroconductivity or electric charges when being irradiated, that is, organo-electronic functional materials, most of the low molecular weight organic compounds are incapable of forming thin film by themselves. Accordingly, when thin film is to be formed with such known low molecular weight organic compounds, they are dispersed in a binder resin (that is, they are diluted with a binder resin), and the resulting dispersion is applied onto a substrate to form thin film. Thus, the conventional organo-electronic functional materials comprised of low molecular weight organic compounds are influenced by the binder resin which forms a matrix, but also they are diluted with the binder resin so that they cannot exhibit sufficiently the properties that they originally possess.

In addition, if the known low molecular weight organic compounds having a photoelectric function form thin film that is relatively stable at normal temperatures with the aid of a binder resin, they have low glass transition temperatures so that the film is poor in heat resistance and is not suitable for practical use. Accordingly, the development of electronic materials that are amorphous at normal temperatures or higher capable of forming amorphous film by themselves has been pushed on with in recent years.

On the other hand, as described in JP-A-6-1972 and JP-A-7-90256, among a variety of electronic devices, an organic electroluminescence element in particular can be driven by a low voltage with high efficiency to emit light at a high luminance, as well as it is a self-emitting device, and hence it can be made thin. Accordingly, in recent years, the investigation to put the organic electroluminescence element to practical use as display devices as well as backlights or illumination devices is pushed forward.

An electroluminescence element is comprised usually of a transparent substrate such as a glass substrate having an anode made of a transparent electrode such as an ITO membrane (indium oxide-tin oxide membrane) laminated thereon, and a hole injecting layer, a hole transporting layer, an emitting layer and a cathode made of a metal electrode laminated on the anode in this order. The anode and the cathode are connected with an external power source. In some cases, the hole injecting layer and the hole transporting layer are formed as a single layer, and in some cases, an electron transporting layer is laminated between the emitting layer and the cathode. Many other layer structures to form organic electroluminescence elements are known, as described in, for example, JP-A-6-1972.

In such an organic electroluminescence element, the hole injecting layer adheres to the anode, and holes are transported from the anode to the emitting layer through the hole injecting layer and the hole transporting layer while electrons are blocked, whereas the electron transporting layer adheres to the cathode, and electrons are transported from the cathode to the emitting layer through the electron transporting layer. Thus, when electrons injected from the cathode and holes injected from the anode recombine in the emitting layer, light is emitted and radiated outside through the transparent electrode (anode) and the transparent substrate. It is known that when a hole injecting layer (and a hole transporting layer) and electron transporting layer are laminated between the electrodes with an emission layer sandwiched therebetween, the resulting organic electroluminescence element has improved emission efficiency.

Such aromatic tertiary amines as 4,4'-bis(N-(3-methylphenyl)-N-phenylamino)biphenyl (TPD) and 4,4'-bis(N-(1-naphthyl)-N-phenylamino))biphenyl (α-NPD) are known as organo-electronic functional materials usable in a hole injecting layer, a hole transporting layer, or a hole injecting/transporting layer, that is, as a hole injecting agent, a hole transporting agent, or a hole injecting/transporting agent, for the conventional organic electroluminescence elements, as described in JP-A-7-90256 and JP-A-5-234681, respectively, for example. However, there is a problem that these aromatic tertiary amines are still insufficient in heat resistance and electric stability. On the other hand, aromatic tertiary amines which have high glass transition temperature have been developed. However, because of their large molecular weights, they need high temperature when being vacuum evaporated, and consequently, there arises a problem that their deterioration and decomposition are accelerated when they are vacuum evaporated.

On the other hand, among aromatic tertiary amines, those which have a carbazole skeleton in the molecule, such as 4,4-di (N-carbazolyl)biphenyl (CBP)and polyvinylcarbazole, have been known, but they have relatively large ionization potential and they have neither good hole injection ability nor hole transporting ability.

In recent years, such a carbazole derivative in which the carbazole skeleton has diarylamino groups at the 3- and 6-position, for example, 3,6-di(diphenylamino)-9-alkylcarbazole and 3,6-di(diphenylamino)-9-phenylcarbazole, have been proposed as a hole transporting agent, as described in Synthetic Metals, 122 (2001), 311-314, Synthetic Metals, 128 (2002), 127-131, and JP-2005- 154421A. However, these carbazole derivatives have still low glass transition temperatures so that it is difficult to use them as a practical heat resistant hole transporting agent.

Furthermore, thin film that is formed by vacuum evaporation of such carbazole derivatives which have heretofore been known is not stable enough thermally and electrically when it is used in an organic electroluminescence element. Accordingly, when the element is heated while it is working, crystallization of the thin film is accelerated and the film is deteriorated. As results, there arises a problem of decrease in luminous efficiency of element, generation and increase of nonluminescent part called dark spots, and the rise of voltage when the element is driven at a fixed current, and there finally arises a problem that the element is destructed.

### Summary of Invention

It is an object of the invention to solve the problems involved in the conventional organic material useful as amorphous electronic material, in particular, as a hole injecting and/or transporting agent, and to provide novel carbazole derivatives which can form stable amorphous film at normal temperature or higher by themselves without aid of binder resin, have a high glass transition temperature or outstanding heat resistance, and hence can be suitably used as an organo-electronic functional material.

In particular, it is an object of the invention to provide novel carbazole derivatives of which use as a hole injecting and/or transporting agent provides an organic electroluminescence element which has high luminance, high efficiency and high durability.

It is a further object of the invention to provide an organic electroluminescence element which has a hole injecting and/or transporting layer comprising a hole injecting and/or transporting agent, respectively, formed of such carbazole derivatives as mentioned above.

The invention provides a carbazole derivative represented by the general formula (I) 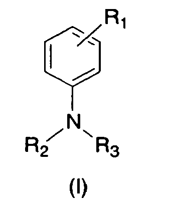 in which R₁ is a hydrogen atom, an alkyl group of 1 to 4 carbon atoms, a cycloalkyl group of 5 to 7 carbon atoms, or an aryl group of 6 to 25 carbon atoms, and R₂ and R₃ are independently an aryl group of 6 to 25 carbon atoms, or any one of first aromatic groups represented by the general formula (II), (III) or (IV) 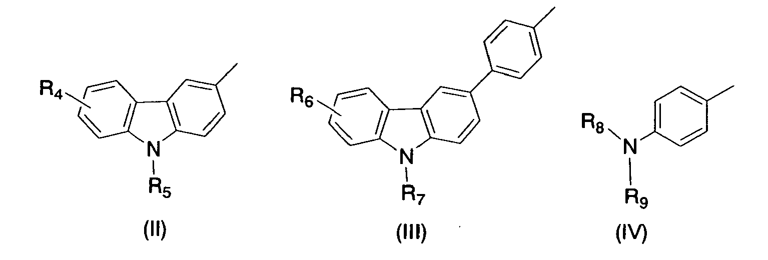 in which R₄ and R₆ are independently a hydrogen atom, an aryl group of 6 to 25 carbon atoms, a diphenylamino group, or a carbazolyl group, and R₅ and R₇ are independently an aryl group of 6 to 25 carbon atoms, a 9-phenyl-3-carbazolyl group, 4-(9-carbazolyl)phenylene group, or 4-diphenylaminophenyl group, and R₈ and R₉ are independently an aryl group of 6 to 25 carbon atoms, or any of second aromatic groups represented by the general formula (V), (VI), or (VII) 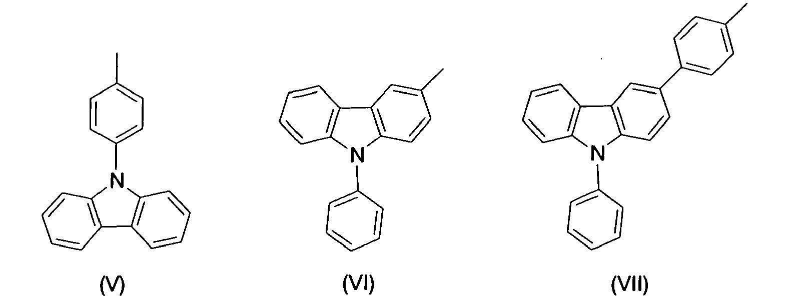 and having at least two carbazole structures in the molecule.

The invention further provides an organo-electronic functional material comprising the carbazole derivative, and as one of preferred embodiments, an organic electroluminescence element comprising such an organo-electronic functional material.

As a particularly preferred embodiment, the invention provides a hole injecting and/or transporting agent comprising the carbazole derivative, as well as an organic electroluminescence element comprising a hole injecting and/or transporting layer comprising such a hole injecting and/or transporting agent.

### Brief Description of the Drawings

Fig. 1 is a sectional view showing an example of an organic electroluminescence element of the invention;
Fig. 2 is a DSC chart of bis(4-(N-phenyl-N-(9-phenyl-3-carbazolyl)amino)phenyl)-p-methylphenylamine (A) of the invention;
Fig. 3 is a TG/DTA chart of bis(4-(N-phenyl-N-(9-phenyl-3-carbazolyl)amino)phenyl)-p-methylphenylamine (A) of the invention;
Fig. 4 is a CV chart of bis(4-(N-phenyl-N-(9-phenyl-3-carbazolyl)amino)phenyl)-p-methylphenylamine (A) of the invention;
Fig. 5 is an infrared absorption spectrum of bis (4-(N-phenyl-N-(9-phenyl-3-carbazolyl)amino)phenyl)-p-methylphenylamine (A) of the invention;
Fig. 6 is a DSC chart of bis(4-(N-phenyl-N-(4-(9-carbazolyl)-phenyl)amino)phenyl)-p-methylphenylamine (B) of the invention;
Fig. 7 is a TG/DTA chart of bis(4-(N-phenyl-N-(4-(9-carbazolyl)-phenyl)amino)phenyl)-p-methylphenylamine (B) of the invention;
Fig. 8 is a CV chart of bis(4-(N-phenyl-N-(4-(9-carbazolyl)-phenyl)amino)phenyl)-p-methylphenylamine (B) of the invention;
Fig. 9 is an infrared absorption spectrum of bis (4-(N-phenyl-N-(4-(9-carbazolyl)phenyl)amino)phenyl)-p-methylphenylamine (B) of the invention;
Fig. 10 is a DSC chart of bis(6-(3-(9'-carbazolyl)-9-phenyl)-carbazolyl)phenylamine (C) of the invention;
Fig. 11 is a TG/DTA chart of bis(6-(3-(9'-carbazolyl)-9-phenyl)-carbazolyl)phenylamine (C) of the invention;
Fig. 12 is a CV chart of bis(6-(3-(9'-carbazolyl)-9-phenyl)-carbazolyl)phenylamine (C) of the invention;
Fig. 13 is an infrared absorption spectrum of bis(6-(3-(9'-carbazolyl)-9-phenyl)carbazolyl)phenylamine (C) of the invention;
Fig. 14 is a DSC chart of bis(6-(3-diphenylamino)-9-phenyl-carbazolyl)phenylamine (D) of the invention;
Fig. 15 is a TG/DTA chart of bis(6-(3-diphenylamino)-9-phenyl-carbazolyl)phenylamine (D) of the invention;
Fig. 16 is a CV chart of bis(6-(3-diphenylamino)-9-phenyl-carbazolyl)phenylamine (D) of the invention;
Fig. 17 is an infrared absorption spectrum of bis (6-(3-diphenylamino)-9-phenylcarbazolyl)phenylamine (D) of the invention;
Fig. 18 is a DSC chart of 3-diphenylamino-6-(9'-(3',6'-bis-(diphenylamino)carbazolyl)-9-phenylamine (E) of the invention;
Fig. 19 is a TG/DTA chart of 3-diphenylamino-6-(9'-(3',6'-bis-(diphenylamino)carbazolyl)-9-phenylamine (E) of the invention;
Fig. 20 is a CV chart of 3-diphenylamino-6-(9'-(3',6'-bis-(diphenylamino)carbazolyl)-9-phenylamine (E) of the invention;
Fig. 21 is an infrared absorption spectrum of 3-diphenylamino-6-(9'-(3',6'-bis(diphenylamino)carbazolyl)-9-phenylamine (E) of the invention;
Fig. 22 is a DSC chart of N,N,N'-tris(9-phenyl-3-carbazolyl)-N'-phenyl-1,4-diaminobenzene (I) of the invention;
Fig. 23 is a TG/DTA chart of N,N,N'-tris(9-phenyl-3-carbazolyl)-N'-phenyl-1,4-diaminobenzene (I) of the invention;
Fig. 24 is a CV chart of N,N,N'-tris(9-phenyl-3-carbazolyl)-N'-phenyl-1,4-diaminobenzene (I) of the invention;
Fig. 25 is an infrared absorption spectrum of N,N,N'-tris-(9-phenyl-3-carbazolyl)-N'-phenyl-1,4-diaminobenzene (I) of the invention;
Fig. 26 is a DSC chart of N-phenyl-N'-(4-(9'-carbazolyl)phenyl-N,N'-bis(9"-phenyl-3"-carbazolyl)-1,4-diaminobenzene (J) of the invention;
Fig. 27 is a TG/DTA chart of N-phenyl-N'-(4-(9'-carbazolyl)-phenyl-N,N'-bis(9"-phenyl-3"-carbazolyl)-1,4-diaminobenzene (J) of the invention;
Fig. 28 is a CV chart of N-phenyl-N'-(4-(9'-carbazolyl)phenyl-N,N'-bis(9"-phenyl-3"-carbazolyl)-1,4-diaminobenzene (J) of the invention;
Fig. 29 is an infrared absorption spectrum of N-phenyl-N'-(4-(9'-carbazolyl)phenyl-N,N'-bis(9"-phenyl-3"-carbazolyl)-1,4-diaminobenzene (J) of the invention;
Fig. 30 is a DSC chart of N,N'-diphenyl-N,N'-bis(4-(9-phenyl-carbazolyl)phenyl)-1,4-diaminobenzene (K) of the invention;
Fig. 31 is a TG/DTA chart of N,N'-diphenyl-N,N'-bis(4-(9-phenylcarbazolyl)phenyl)-1,4-diaminobenzene (K) of the invention;
Fig. 32 is a CV chart of N,N'-diphenyl-N,N'-bis(4-(9-phenyl-carbazolyl)phenyl)-1,4-diaminobenzene (K) of the invention;
Fig. 33 is an infrared absorption spectrum of N,N'-diphenyl-N,N'-bis(4-(9-phenylcarbazolyl)phenyl)-1,4-diaminobenzene (K) of the invention;
Fig. 34 is a DSC chart of 6'-(N-carbazolyl)-bis(9-phenyl-3-carbazolyl)phenylamine (M) of the invention;
Fig. 35 is a CV chart of 3-(9-phenylcarbazolyl)-3'-(6'-(9"-carbazolyl)-9'-phenyl)carbazolyl)phenylamine (M) of the invention;
Fig. 36 is an infrared absorption spectrum of 3-(9-phenyl-carbazolyl)-3'-(6'-(9"-carbazolyl)-9'-phenyl)carbazolyl)phenylamine (M) of the invention;
Fig. 37 is a DSC chart of 3-carbazolyl-3'-(6'-(9"-carbazolyl)-9'-phenylcarbazolyl)-p-methylphenylamine (N) of the invention;
Fig. 38 is a CV chart of 3-carbazolyl-3'-(6'-(9"-carbazolyl)-9'-phenylcarbazolyl)-p-methylphenylamine (N) of the invention; and
Fig. 39 is an infrared absorption spectrum of 3-carbazolyl-3'-(6'-(9"-carbazolyl)-9'-phenylcarbazolyl)-p-methylphenyl amine (N) of the invention.

### Description of Embodiments

The novel carbazole derivative of the invention is represented by the general formula (I) 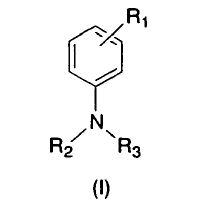 in which R₁ is a hydrogen atom, an alkyl group of 1 to 4 carbon atoms, a cycloalkyl group of 5 to 7 carbon atoms, or an aryl group of 6 to 25 carbon atoms, and R₂ and R₃ are independently an aryl group of 6 to 25 carbon atoms, or any one of first aromatic groups represented by the general formula (II), (III) or (IV) 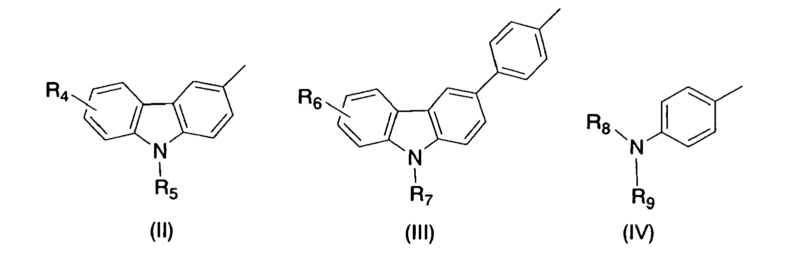 in which R₄ and R₆ are independently a hydrogen atom, an aryl group of 6 to 25 carbon atoms, a diphenylamino group, or a 9-carbazolyl group, and R₅ and R₇ are independently an aryl group of 6 to 25 carbon atoms, a 9-phenyl-3-carbazolyl group, 4-(9-carbazolyl)phenylene group, or 4-diphenylaminophenyl group, and R₈ and R₉ are independently an aryl group of 6 to 25 carbon atoms, or any of second aromatic groups represented by the general formula (V), (VI), or (VII) 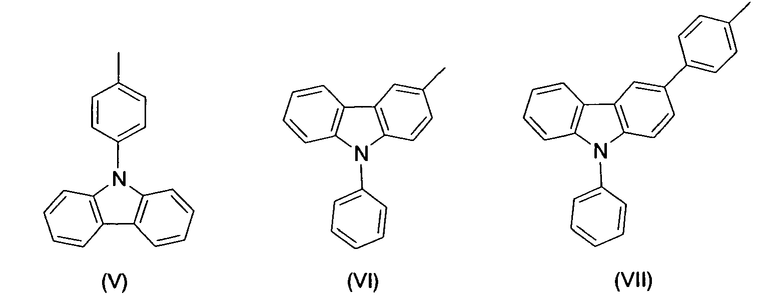 and having at least two carbazole structures in the molecule.

In the carbazole derivatives represented by the above general formula (I), the alkyl group of 1-4 carbon atoms is preferably a methyl group or an ethyl group; the cycloalkyl group of 5-7 carbon atoms is preferably a cyclohexyl group; and the aryl group of 6-25 carbon atoms is preferably a phenyl group, a naphthyl group, an anthryl group, or a phenanthryl group, and more preferably a phenyl group or a naphthyl group, and most preferably a phenyl group.

Therefore, in the carbazole derivatives represented by the above general formula (I), R₁ is preferably a hydrogen atom, a methyl group, an ethyl group, a cyclohexyl group, or a phenyl group, and most preferably a hydrogen atom or a methyl group.

In the carbazole derivatives represented by the above general formula (I), it is preferred that R₂ and R₃ are independently a phenyl group or any of first aromatic groups represented by the general formula (II), (III) or (IV) wherein R₄ and R₆ are independently a hydrogen atom, a phenyl group, a diphenylamino group or 9-carbazolyl group; R₅ and R₇ are independently a phenyl group, a 9-phenyl-3-carbazolyl group, 4-(9-carbazolyl)phenylene group or 4-diphenylaminophenyl group; and R₈ and R₉ are independently a phenyl group or any of second aromatic groups represented by the general formula (V), (VI), or (VII).

According to the invention, in particular, it is preferred that the above-mentioned R₄ and R₆ are independently a hydrogen atom, a phenyl group, a diphenylamino group, or 9-carbazolyl group, and the above-mentioned R₅ and R₇ are independently a phenyl group, a 9-phenyl-3-carbazolyl group, 4-(9-carbazolyl)phenylene group, or 4-diphenylaminophenyl group.

It is preferred that the above-mentioned R₈ and R₉ are independently a phenyl group or any of the second aromatic groups represented by the general formula (V), (VI) or (VII).

Further according to the invention, the novel carbazole derivatives of the invention have at least two carbazole structures in the molecule.

Therefore, examples of preferred carbazole derivative of the invention are shown below as (A) to (N).

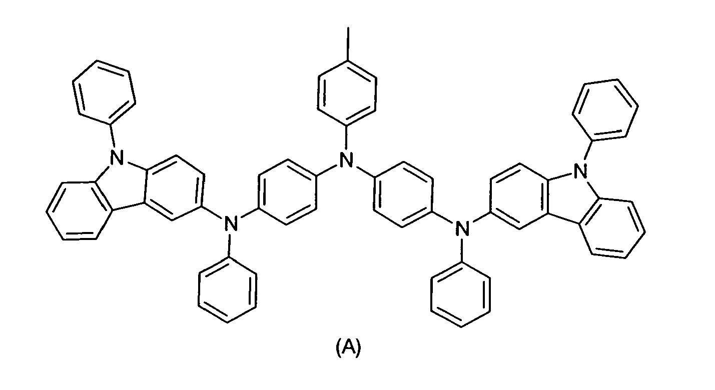

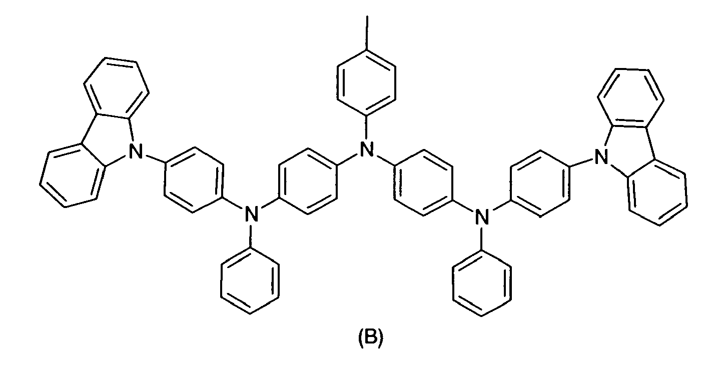

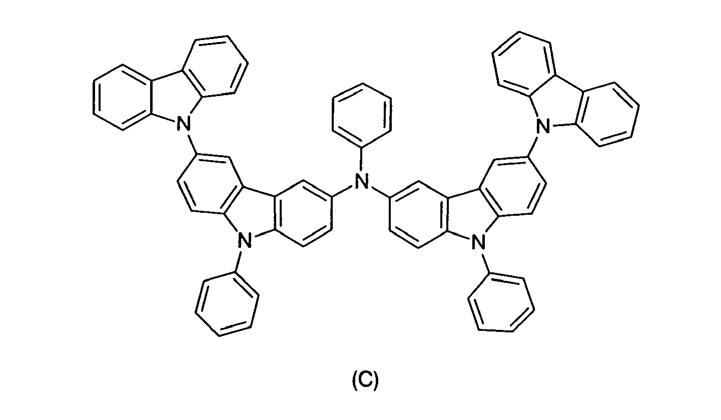

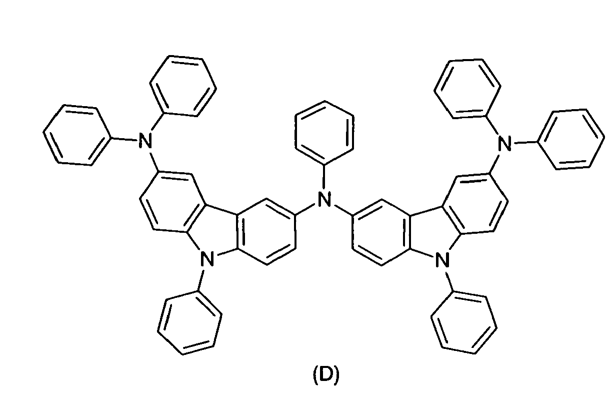

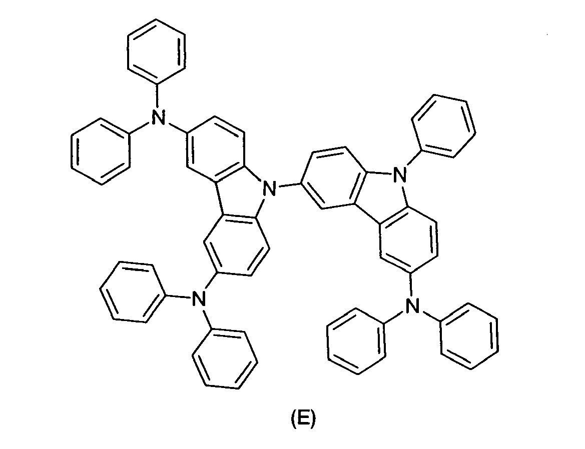

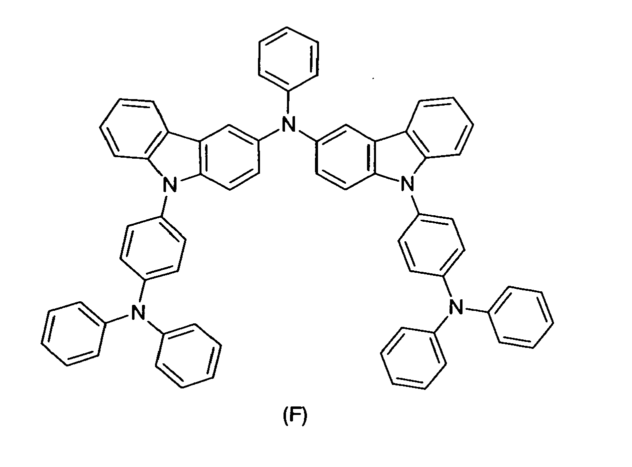

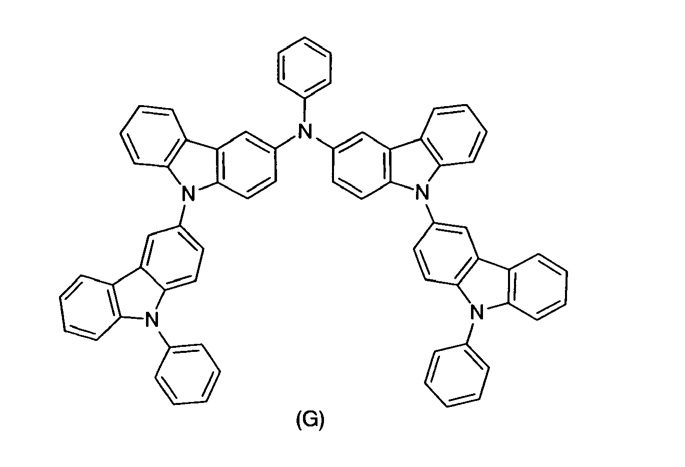

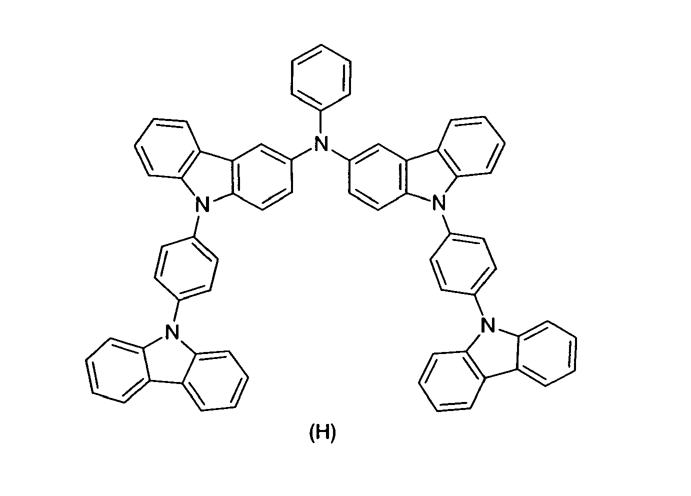

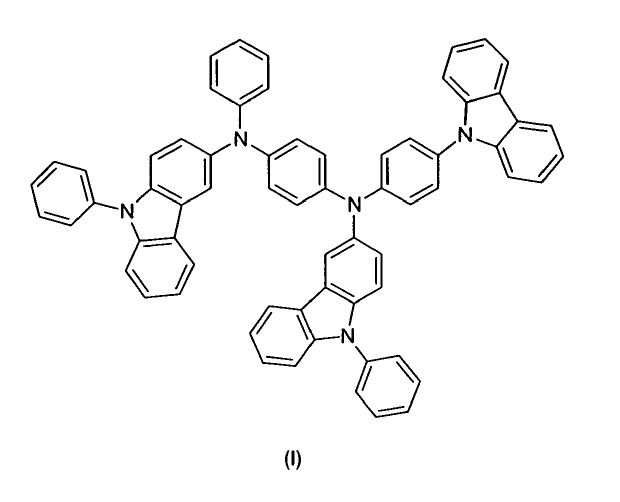

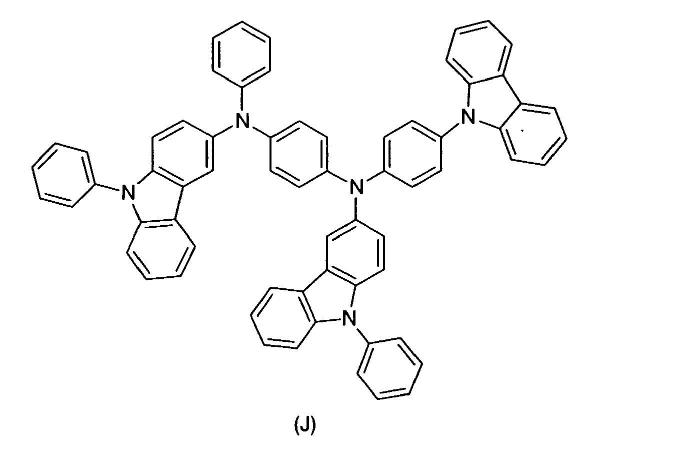

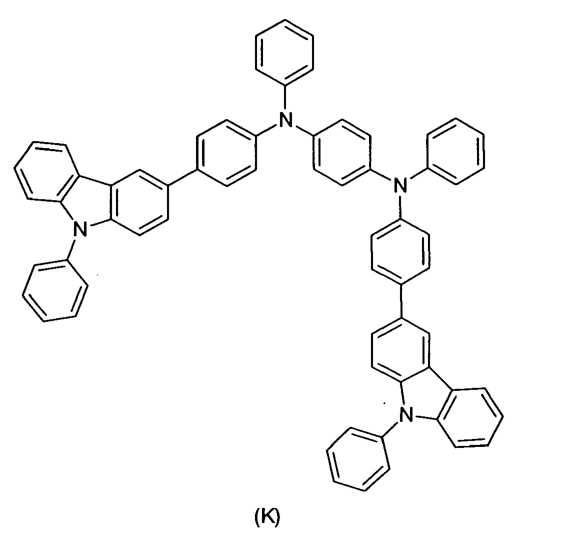

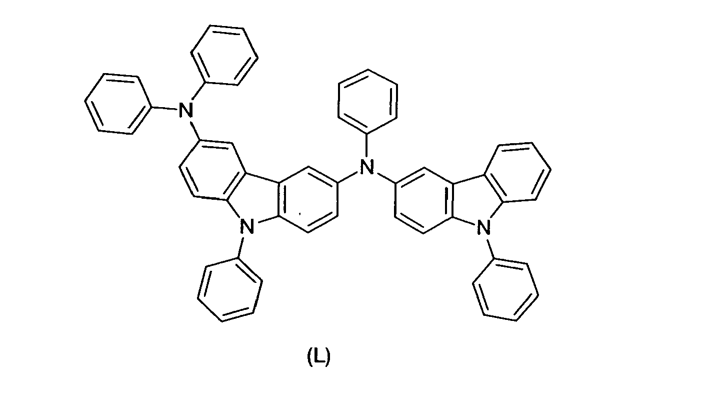

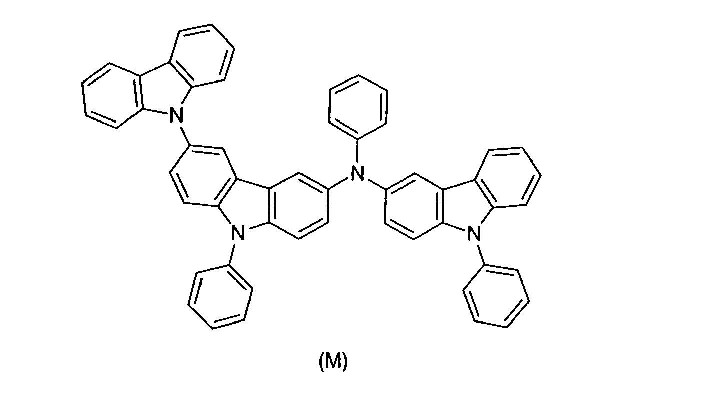

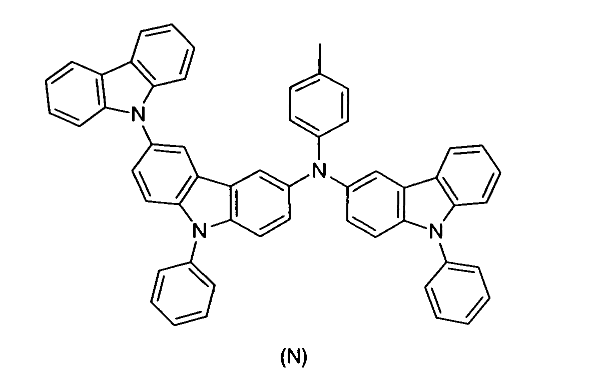

The carbazole derivative of the invention has a very high glass transition temperature and a decomposition temperature, and hence it is cable of forming stable amorphous film by itself, that is, without aid of binder resin, at normal temperature or higher, and in addition, it has excellent heat resistance. Therefore, by way of examples, when a hole injecting layer wherein the carbazole derivative of the invention is used as a hole injecting agent is formed between an anode and a hole transporting layer, or when a hole transporting layer wherein the carbazole derivative of the invention is used as a hole transporting agent is formed between a hole injecting layer and an emitting layer, or when a hole injecting/transporting layer wherein the carbazole derivative of the invention is used as a hole injecting/transporting agent is formed between a cathode and an emitting layer, an organic electroluminescence element which is excellent in durability and efficiency can be obtained. However, needless to say, the carbazole derivative of the invention may be used to form a thin film as a hole injecting and/or transporting layer using a binder resin.

As mentioned above, the carbazole derivative of the invention can be used as a hole injecting agent to form a hole injecting layer in an organic electroluminescence element. Accordingly, it can also be used as a hole transporting agent to form a hole transporting layer in an organic electroluminescence element, as a hole transporting layer has the same function as a hole injecting layer. Further, it can also be used as a hole injecting/transporting agent when a hole injecting layer and a hole transporting layer are formed as a single layer. Namely, the carbazole derivative of the invention can be used as a hole injecting and/or transporting agent, and such a hole injecting and/or transporting agent can be used in order to form a hole injecting and/or transporting layer in an organic electroluminescence element. The carbazole derivative of the invention can also be used as a host agent in an emission layer of an organic electroluminescence element.

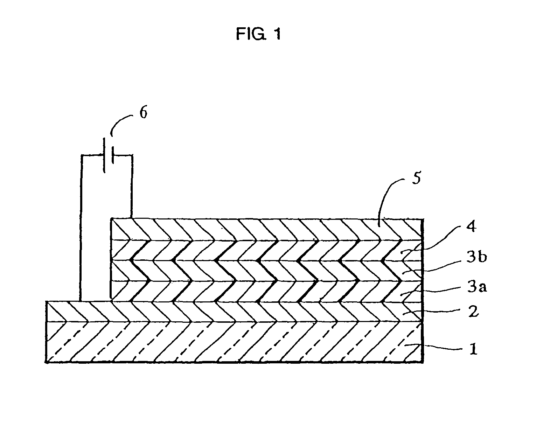

As a preferred example of an electroluminescence element according to the invention is shown in Fig. 1, it comprises a transparent substrate 1 made of glass, for example, having an anode 2 made of a transparent electrode such as an ITO membrane (indium oxide-tin oxide membrane) laminated thereon, and a hole injecting layer 3a, a hole transporting layer 3b, an emitting layer 4 and a cathode 5 made of a metal or a compound thereof laminated on the anode in this order. The anode and the cathode are connected with an external power source 6. In such an organic electroluminescence element as mentioned above, holes are readily injected from the anode into the emitting layer through the hole injecting layer and the hole transporting layer so that the organic electroluminescence element can be driven at a low electric voltage. Electrons are injected into the emitting layer from the cathode. The electrons injected from the cathode and the holes injected from the anode recombine in the emitting layer, and light is emitted and radiated outside through the transparent electrode (anode) and the transparent substrate.

According to the invention, in some cases, an electron transporting layer may be laminated between the emitting layer and the cathode, and a blocking layer may be formed in order to prevent excessive holes from passing through the emitting layer towards the cathode. The organic electroluminescence element is not specifically limited in layer structure.

Since the carbazole derivative of the invention can form amorphous film by itself, a hole injecting layer can be formed by vacuum evaporation of the carbazole derivative of the invention onto the transparent electrode using a vacuum evaporation device. The thickness of hole injecting layer is usually in the range of 10 nm to 200 nm, preferably in the range of 20 nm to 80 nm. The carbazole derivative of the invention can also be vacuum evaporated onto a hole injecting layer to form a hole transporting layer. The thickness of hole transporting layer is also usually in the range of 10 nm to 200 nm, preferably in the range of 20 nm to 80 nm. Further, a hole injecting and/or transporting layer in the form of single layer may be formed on the transparent electrode.

However, according to the invention, the carbazole derivative of the invention may be dissolved in an appropriate organic solvent, if necessary, together with a binder resin, to prepare a solution, and then the solution may be coated on an anode by a spin coat method and dried to form a hole injecting and/or transporting layer. In this case also, the thickness of hole injecting and/or transporting layer is the same as that mentioned above.

In this way, a hole injecting layer is formed with the carbazole derivative of the invention, and a hole transporting layer is formed with α-NPD etc. by a conventional method on the hole injecting layer, and then an emitting layer and a cathode are laminated on the hole transporting layer, thereby an organic electroluminescence element is obtained. In the same manner, a hole transporting layer is formed with the carbazole derivative of the invention on a hole injecting layer, and then an emitting layer and a cathode are formed on the hole injecting layer, thereby an organic electroluminescence element is obtained.

When an organic electroluminescence element of the invention is so formed as to contain a hole injecting and/or transporting layer formed using the carbazole derivative of the invention, the other members, for example, a transparent substrate, a common hole injecting and/or transporting layer which may be used in combination with the hole injecting and/or transporting layer of the invention, an anode, an emitting layer, an electron transporting layer and electrodes may be made of any conventionally known materials. For example, an anode may be preferably made of indium oxide-tin oxide (ITO), and a cathode may be made of a metal such as aluminum, magnesium, indium or silver, or an alloy of these metals, such as A1-Mg alloy, Ag-Mg alloy, or lithium fluoride. A transparent substrate is usually made of glass.

For example, such a conventionally known low molecular weight organic compound as α-NPD (4,4'-bis(N-(1-naphthyl)-N-phenylamino)-biphenyl) and TPD (4,4'-bis(3-methylphenyl)-N-phenylamino)biphenyl) are used as a common hole transporting agent, while copper phthalocyanine etc. are used as a common hole injecting agent. The thickness of any of the layers formed as mentioned above is usually in the range of 10 nm to 200 nm.

The emitting layer is usually formed of tris(8-quinolinol) aluminum (Alq₃) and has a thickness in the range of 10 nm to 200 nm. When the organic electroluminescence element contains an electron transporting layer, the layer has also a thickness in the range of 10 nm to 200 nm.

The carbazole derivative of the invention is not specifically limited in use, and it can be suitably used as a hole injecting agent, a hole transporting agent, and a host agent in an emitting layer for an organic electroluminescence element, and in addition, as an organic semiconductor for a solar cell and a charge transporting material for an electrophotographic device.

### Industrial Applicability

The invention provides the novel carbazole derivatives represented by the general formula (I). The carbazole derivatives not only form stable amorphous film at normal temperature or higher, but also have a higher glass transition temperature and hence a higher heat resistance than the aromatic tertiary amines or carbazole derivatives which have hitherto been known. Accordingly, the novel carbazole derivatives of the invention are suitably used in an organic electroluminescence element as a hole injecting and/or transporting agent which has ability to form highly amorphous film and has excellent heat resistance. The use of the carbazole derivatives of the invention as a hole injecting and/or transporting agent in this way provides an organic electroluminescence element which can be driven at a lower voltage at a higher luminance and which is more durable than the conventional organic electroluminescence elements.

### Examples

The invention will now be explained with reference to examples. However, the invention is not limited to these examples.

### Example I (Synthesis and Properties of Carbazole Derivatives)

### Example 1

Bis (4-(N-phenyl-N-(9-phenyl-3-carbazolyl)amino)phenyl)-p-methylphenylamine (A) was synthesized according to the following scheme.

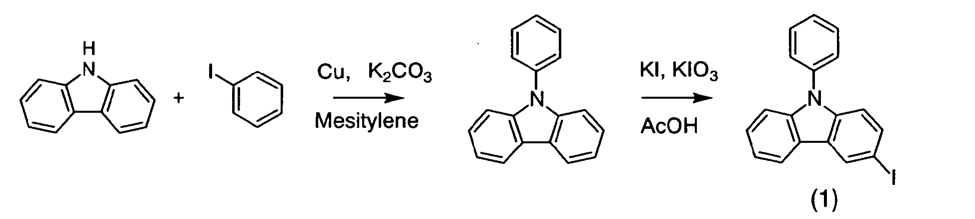

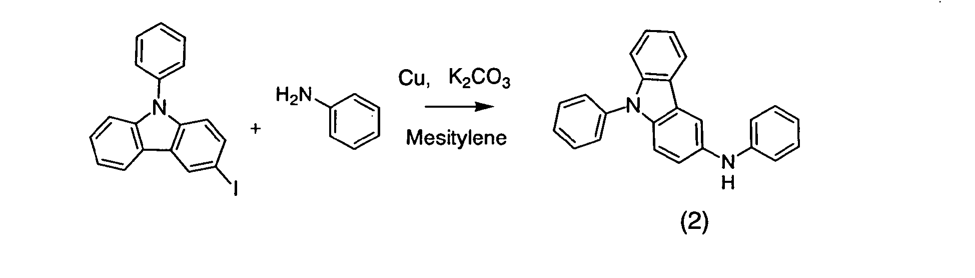

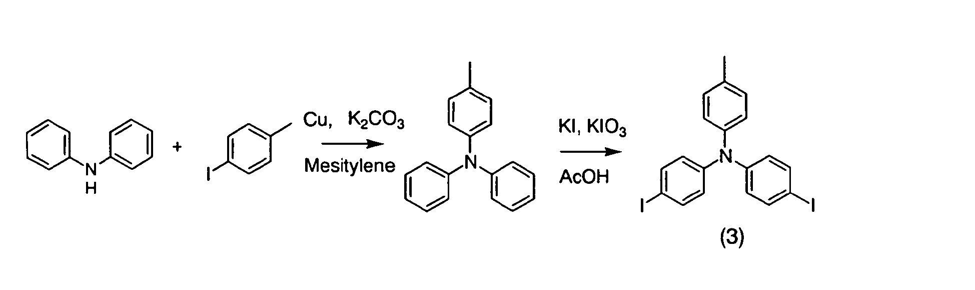

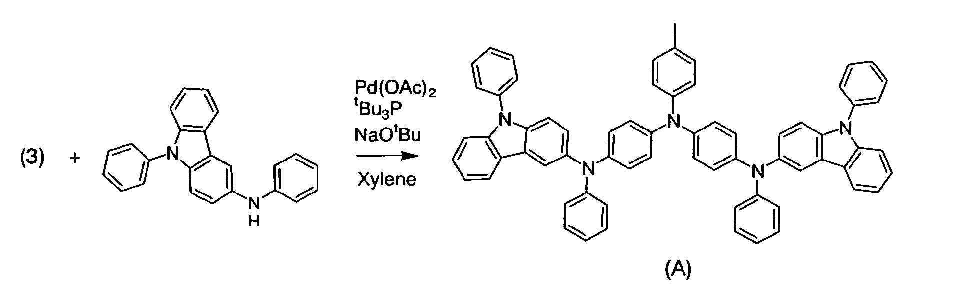

37.0g of iodobenzene, 25.0 g of carbazole, 50.5g of potassium carbonate and 15 g of copper powder were added to mesitylene, and were reacted under reflux for three hours under a nitrogen atmosphere. After completion of the reaction, the resulting reaction mixture was extracted with toluene, and fractionated by silica gel column chromatography using a mixed solvent composed of toluene/hexane (volume ratio of 1/1) to separate the desired compound. The obtained solid was recrystallized from hexane to provide 31.5 g of 9-phenylcarbazole.

The 9-phenylcarbazole was dissolved in 300mL of acetic acid, and then 7 g of potassium iodide and 18 g of potassium iodate were added to the resultant. The resulting solution was reacted at a temperature of 80°C for two hours. After completion of the reaction, unreacted iodine in the resulting reaction mixture was reduced with aqueous solution of sodium thiosulfate, and then the reaction mixture was concentrated, followed by purifying the resulting concentrate by silica gel column chromatography. The resulting viscous liquid was subjected to crystallization from hexane to provide 20.5 g of 3-iodo-9-phenylcarbazole (1) as white solid.

20 g of 3-iodo-9-phenylcarbazole was reacted with 10 g of aniline at a temperature of 165°C in a solvent of mesitylene for five hours in the presence of 35 g of potassium carbonate and 10 g of copper powder. After completion of the reaction, the resulting reaction mixture was dissolved in toluene, the resulting solution was filtered to remove inorganic materials therefrom, and the resultant was subjected to fractionation by silica gel column chromatography using a mixed solvent composed of toluene/hexane as eluent to separate the desired compound. The obtained solid was recrystallized from hexane to provide 12.3 g of phenyl-9-phenyl-3-carbazolylamine (2) as white solid.

50 g of diphenylamine, 64.3 g of 4-iodotoluene, 100 g of potassium carbonate, and 20 g of copper powder were added to mesitylene, and were refluxed for seven hours. Toluene was added to the resulting reaction mixture to dissolve the desired product. The resulting solution was concentrated, filtered with a silica gel column, and then crystallization was carried out from a mixed solvent of toluene/hexane to provide diphenyl-p-tolylamine.

The diphenyl-p-tolylamine was iodinated using 35 g of potassium iodide and 150 g of potassium iodate in acetic acid. The resulting reaction mixture was fractionated by silica gel column chromatography using toluene as an eluent, followed by recrystallization from toluene/hexane to provide 120 g of bis(4-iodophenyl)-p-tolylamine (3) as pale yellow needle crystals.

8 g of bis(4-iodophenyl)-p-tolylamine and 12 g of phenyl-9-phenyl-3-carbazolylamine were added to xylene solution of 5.1 g of sodium t-butoxide, 0.01g of palladium acetate and 0.4 g of tri-t-butylphosphine, and were reacted at a temperature of 120°C for six hours under a nitrogen atmosphere. After completion of the reaction, the resulting reaction mixture was cooled to room temperature, and 50mL of water was added thereto and stirred, and then a mixture of toluene/water was added to the resulting mixture. The resulting organic layer was separated and concentrated. The resulting viscous liquid was fractionated by silica gel column chromatography to separate the desired product, which was then recrystallized from toluene/ethanol, and subjected to sublimation purification, thereby providing 10.5 g of bis(4-(N-phenyl-N-(9-phenyl-3-carbazolyl)amino)-phenyl)-p-methylphenylamine (A) as pale yellow solid.

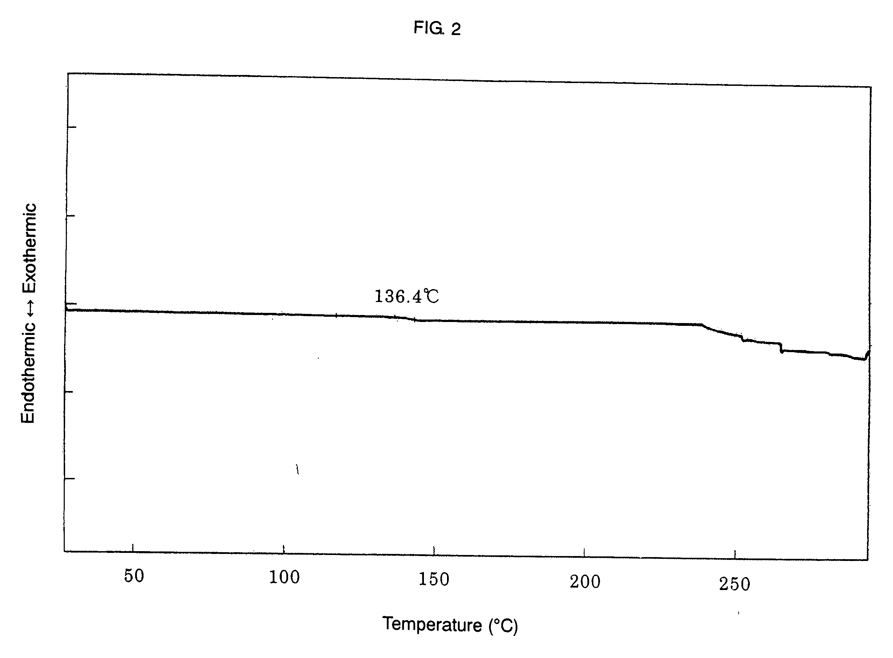

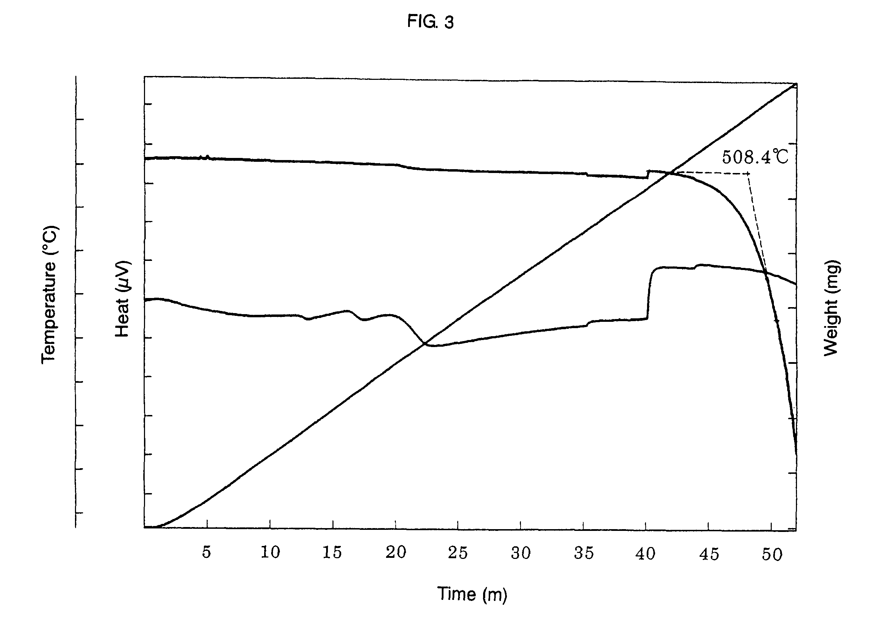

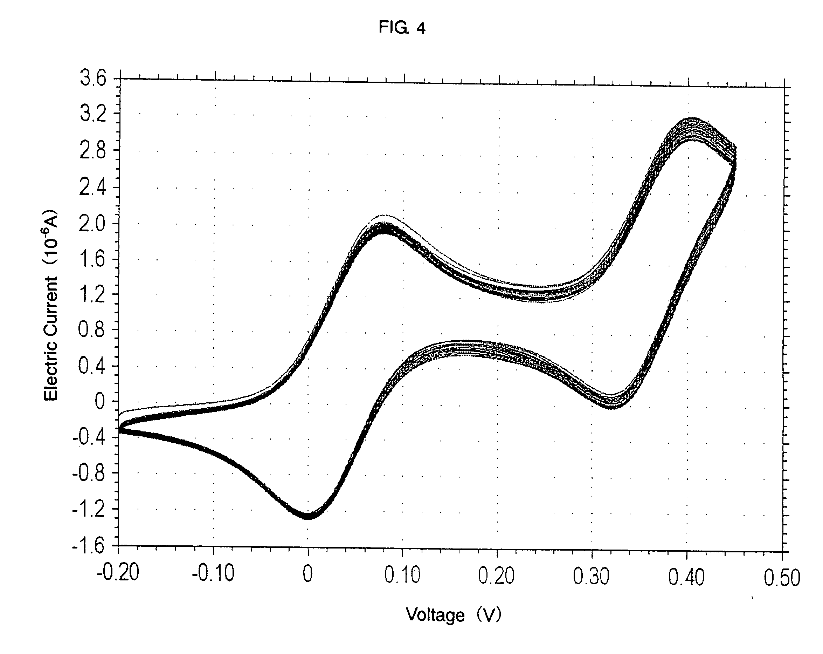

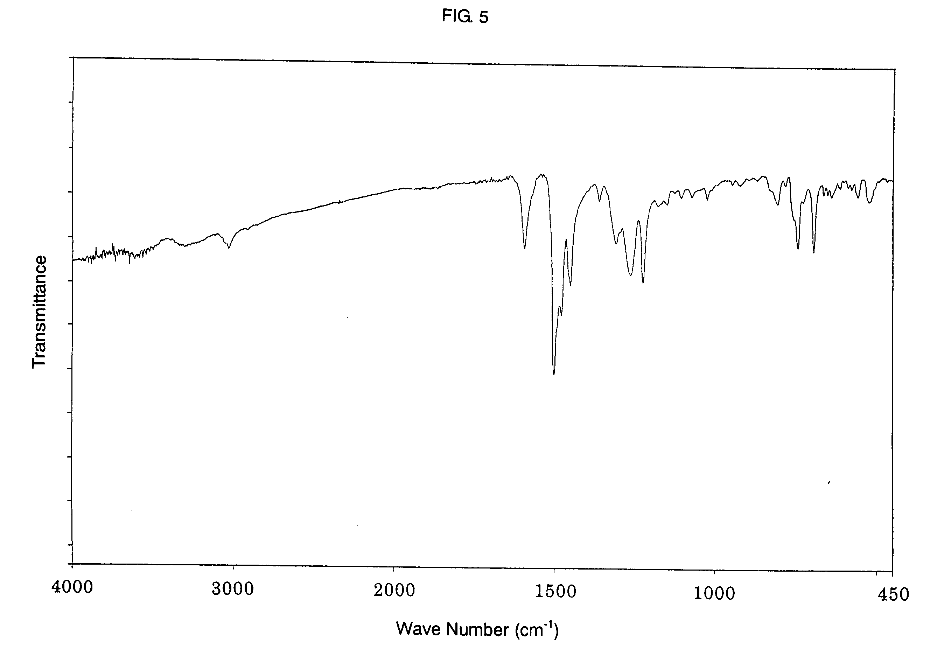

The molecular weight (M⁺ (m/e)) measured by mass analysis and the results of elemental analysis are shown in Table 1. The ionization potential, the glass transition temperature measured by differential scanning calorimetric analysis (DSC), the decomposition temperature measured by thermogravimetric measurement/differential calorimetry (TG/DTA), and the oxidation potential (vs Ag/Ag⁺) measured by cyclic voltammetry (CV) are also shown in Table 1. The DSC chart is shown in Fig. 2, the TG/DTA chart is shown in Fig. 3, the CV chart is shown in Fig. 4, and the infrared absorption spectrum (KBr tablet method) is shown in Fig. 5.

### Example 2

Bis(4-(N-phenyl-N-(4-(9-carbazolyl)phenyl)amino)phenyl)-p-methylphenylamine (B) was synthesized according to the following scheme.

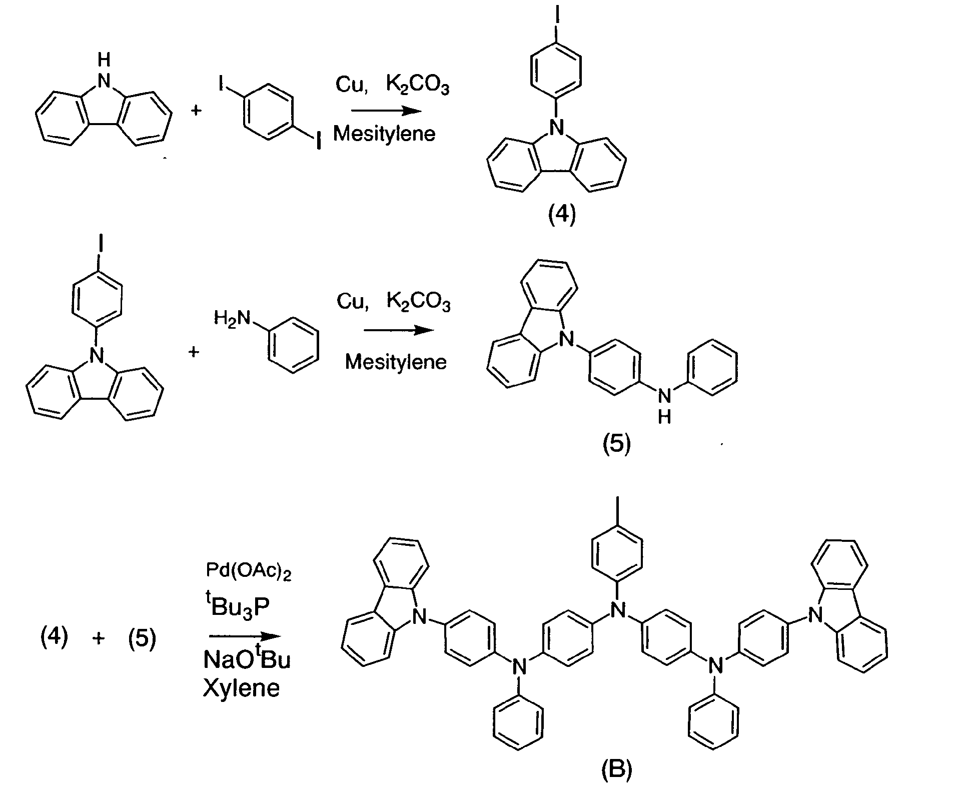

100 g of 1,4-diiodobenzene, 50 g of carbazole, 80 g of potassium carbonate, and 20 g of copper powder were added to mesitylene, and were reacted under reflux for eight hours. The desired product was extracted with toluene from the resulting reaction mixture, and purified by silica gel column chromatography, followed by further purification by recrystallization from toluene, to provide 87.2 g of 9-(4-iodophenyl)carbazole (4).

In place of 3-iodo-9-phenylcarbazole, 9-(4-iodophenyl)-carbazole was reacted with aniline, and otherwise in the same manner as in Example 1, phenyl-4-(9-carbazolyl)phenylamine (5) was obtained. This was reacted with 9-(4-iodophenyl)carbazole (4) in the same manner as in Example 1, to provide bis(4-(N-phenyl-N-(4-(9-carbazolyl)phenyl)amino)phenyl)-p-methylphenylamine (B).

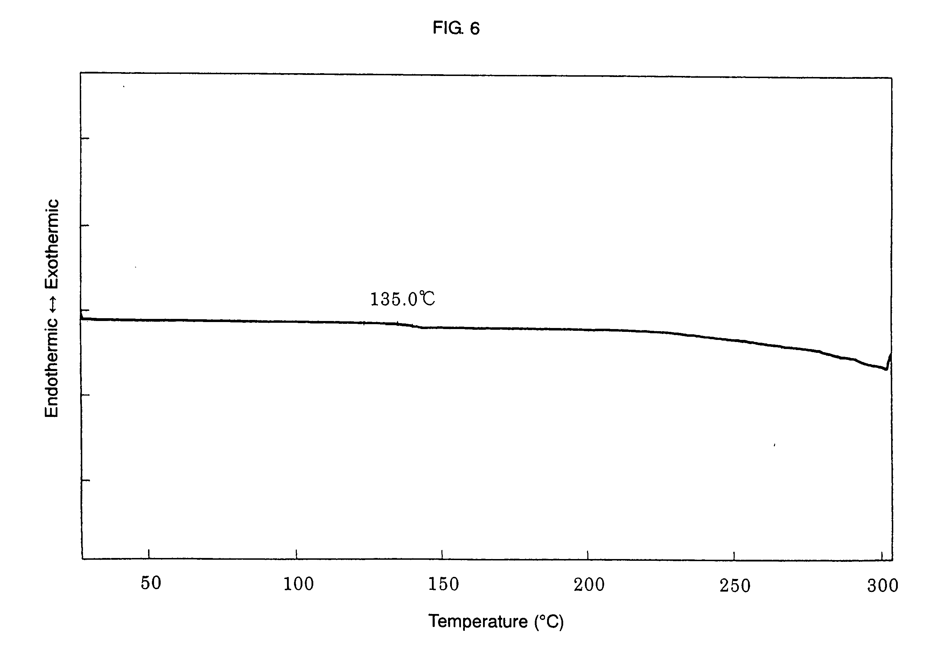

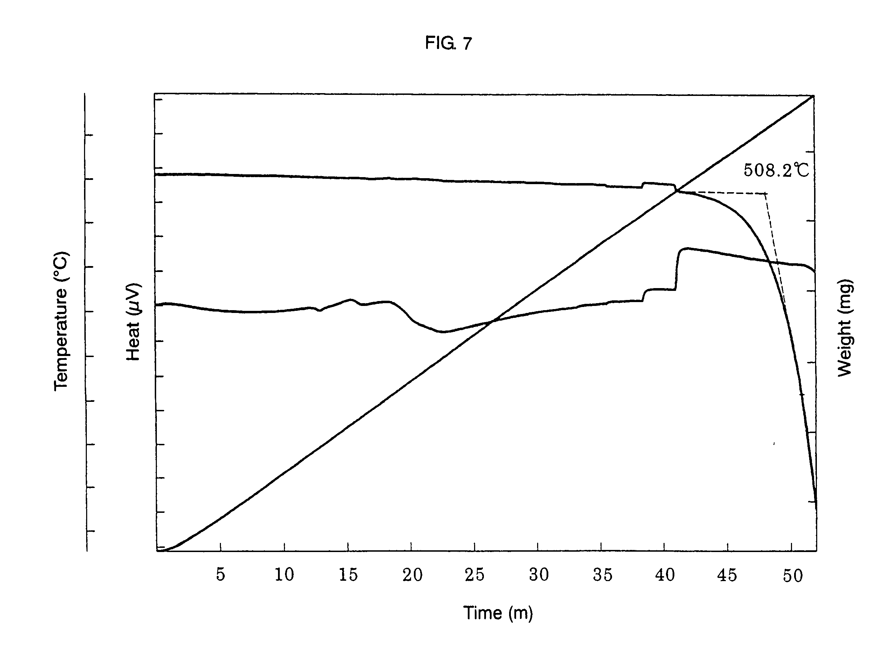

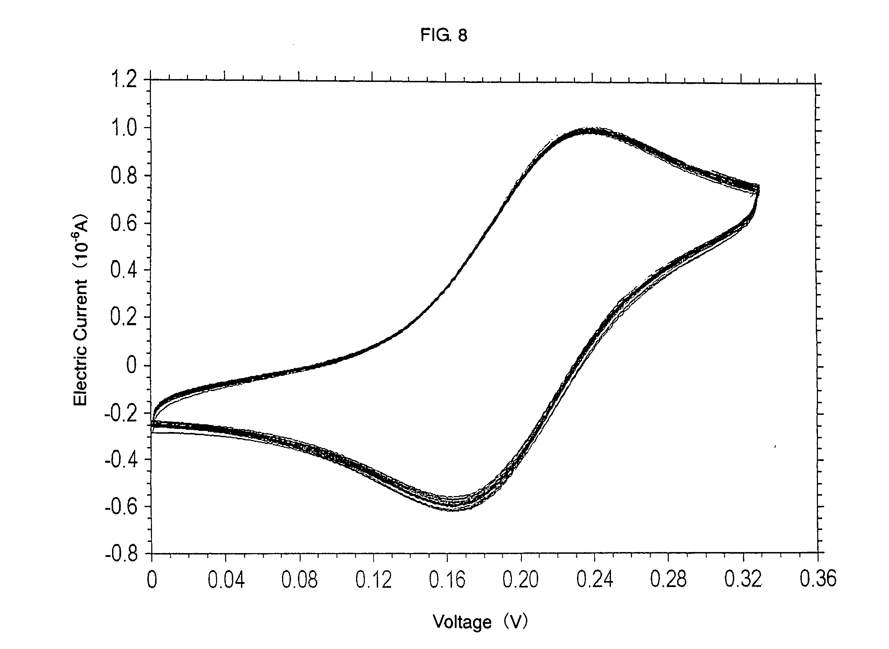

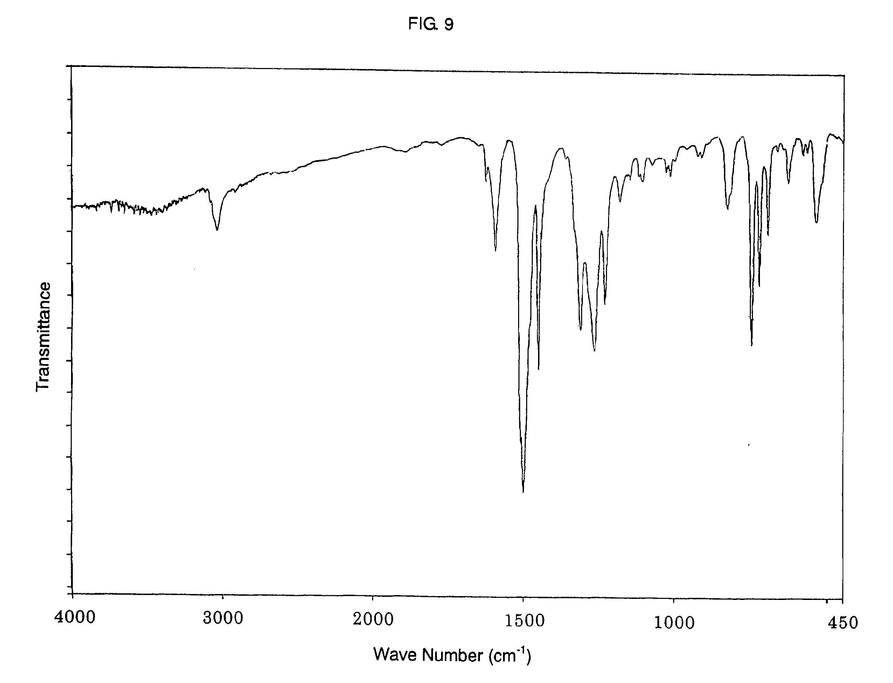

The molecular weight (M⁺ (m/e)) measured by mass analysis and the results of elemental analysis are shown in Table 1. The ionization potential, the glass transition temperature measured by differential scanning calorimetric analysis (DSC), the decomposition temperature measured by thermogravimetric measurement/differential calorimetry (TG/DTA), and the oxidation potential (vs Ag/Ag⁺) measured by cyclic voltammetry (CV) are shown in Table 2. The DSC chart is shown in Fig. 6, the TG/DTA chart is shown in Fig. 7, the CV chart is shown in Fig. 8, and the infrared absorption spectrum (KBr tablet method) is shown in Fig. 9.

### Example 3

Bis (6-(3-(9'-carbazolyl)-9-phenyl)carbazolyl)phenylamine (C) was synthesized according to the following scheme.

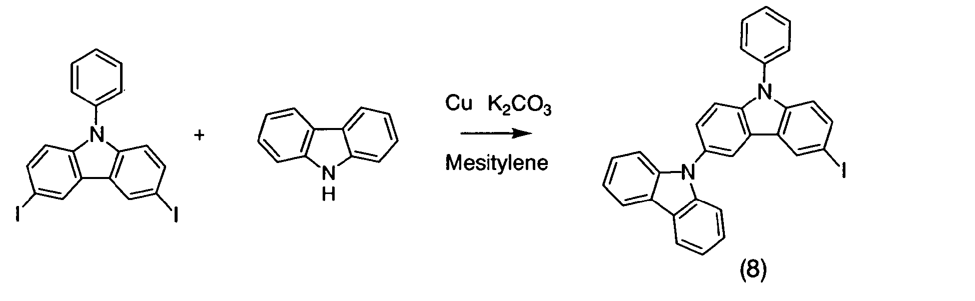

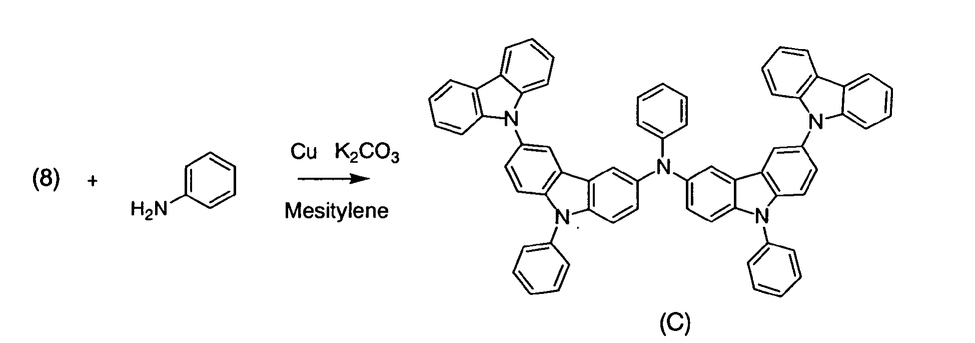

In place of diphenylamine, carbazole was reacted with 3,6-diiodo-9-phenylcarbazole, and otherwise in the same manner as in Example 1, 3-(9-carbazolyl)-6-iodo-9-phenylcarbazole (8) was obtained. This was reacted with aniline in the same manner as in Example 1 to provide bis(6-(3-(9'-carbazolyl)-9-phenyl)carbazolyl)phenylamine (C).

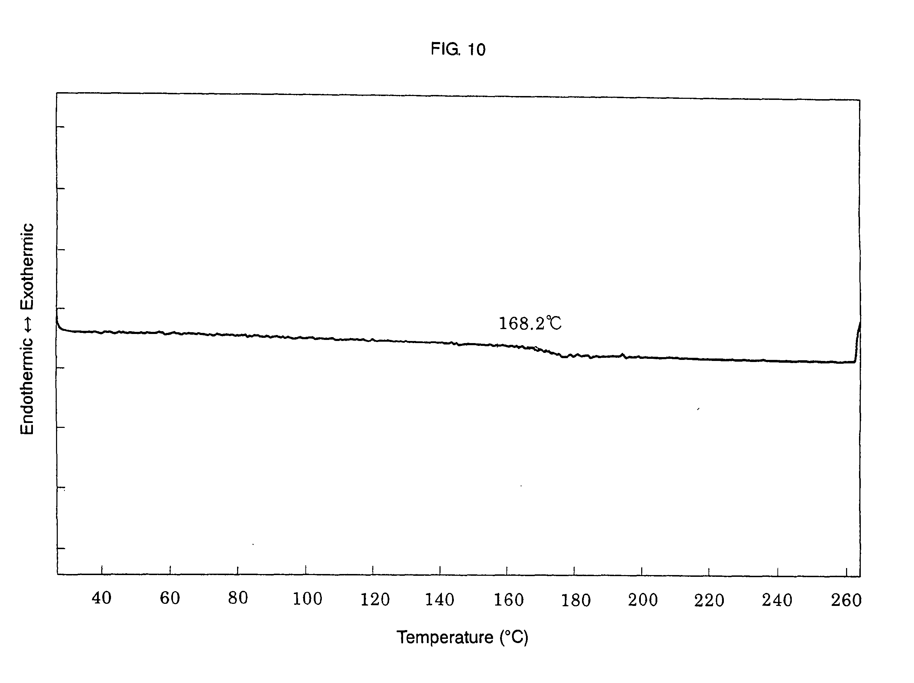

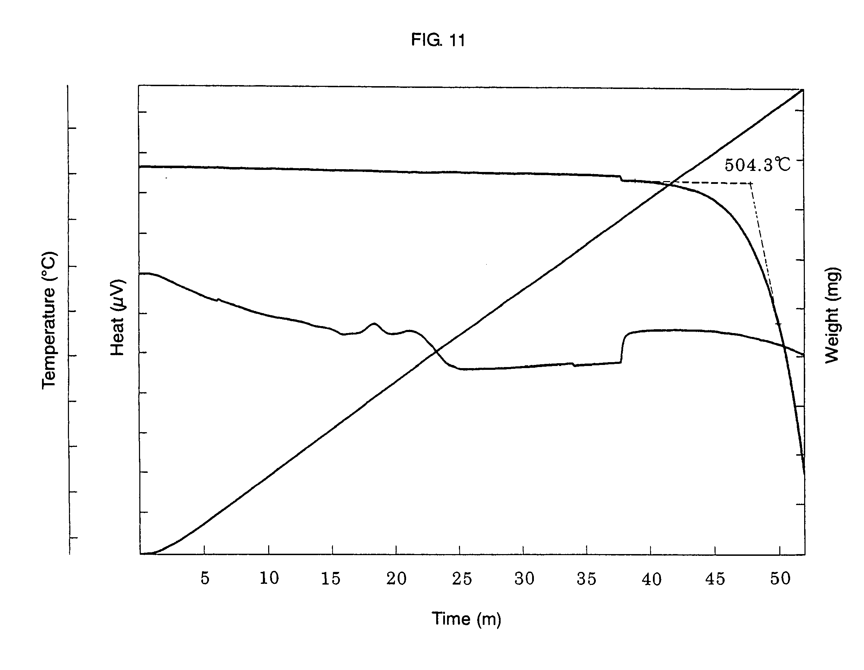

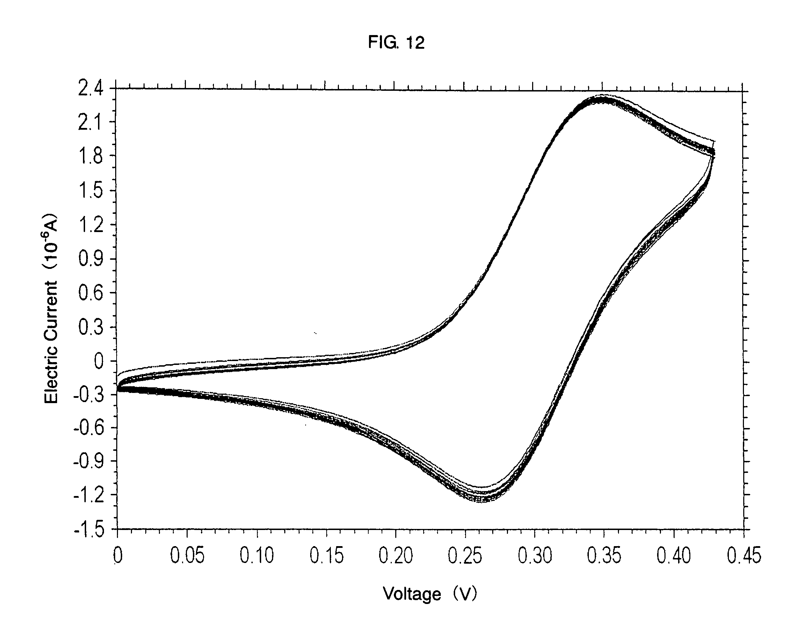

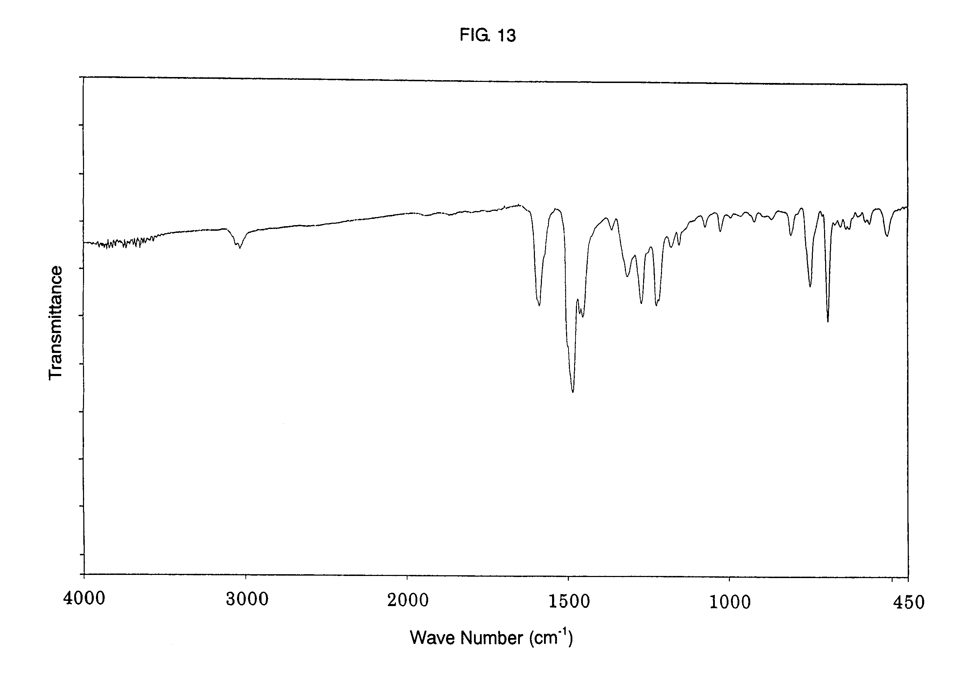

The molecular weight (M⁺ (m/e)) measured by mass analysis and the results of elemental analysis are shown in Table 1. The ionization potential, the glass transition temperature measured by differential scanning calorimetric analysis (DSC), the decomposition temperature measured by thermogravimetric measurement/differential calorimetry (TG/DTA), and the oxidation potential (vs Ag/Ag⁺) measured by cyclic voltammetry (CV) are shown in Table 2. The DSC chart is shown in Fig. 10, the TG/DTA chart is shown in Fig. 11, the CV chart is shown in Fig. 12, and the infrared absorption spectrum (KBr tablet method) is shown in Fig. 13.

### Example 4

Bis (6-(3-diphenylamino)-9-phenylcarbazolyl)phenylamine (D) was synthesized according to the following scheme.

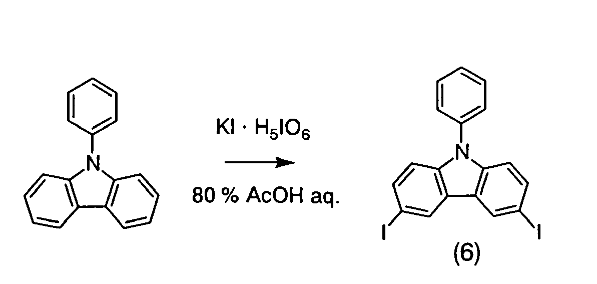

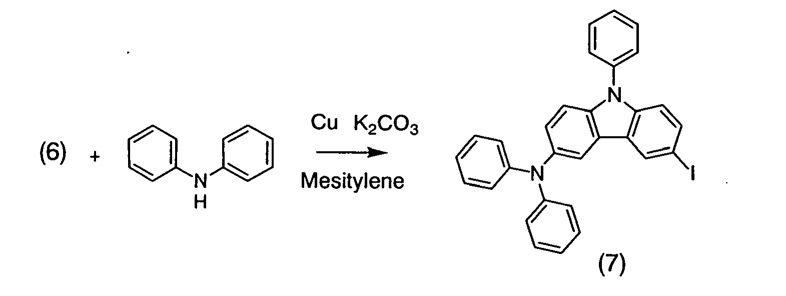

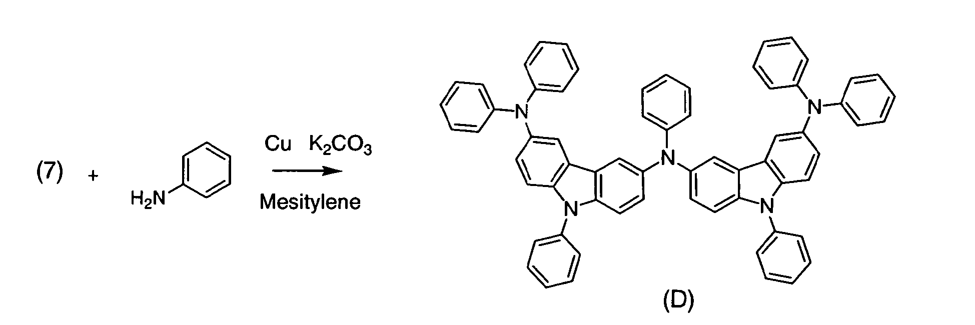

30.0 g of 9-phenylcarbazole was dissolved in 50mL of acetic acid at a temperature of 80°C. 81.5 g of potassium iodide and 53.5 g of potassium orthoperiodate dihydrate were added to the resultant in several portions. After addition of the whole quantity, the resultant mixture was reacted with stirring for one hour. After completion of the reaction, the resulting reaction mixture was cooled to room temperature. The reaction mixture was extracted with a mixture of ethyl acetate and 10% aqueous solution of sodium thiosulfate, and the obtained organic phase was concentrated to provide viscous liquid. The viscous liquid was purified using a mixed solvent of toluene/hexane by alumina column chromatography, thereby 32.7 g of 3,6-diiodo-9-phenylcarbazole (6) was obtained.

30 g of 3,6-diiodo-9-phenylcarbazole was reacted with 10 g of diphenylamine in the presence of 40 g of potassium carbonate and 10g of copper powder in a solvent of mesitylene. The resulting reaction mixture was fractionated by silica gel column chromatography to provide 13 g of 3-(6-iodo-9-phenyl)carbazolyldiphenylamine (7).

7.2 g of 3-(6-iodo-9-phenyl)carbazolyldiphenylamine, 1.5 g of aniline, 5 g of potassium carbonate and 5 g of copper powder were added to mesitylene, and were refluxed for 12 hours under a nitrogen atmosphere. The resulting reaction mixture was dropwise added into ethanol to provide yellow solid. The solid was dissolved in toluene, and the resulting solution was subjected to silica gel column chromatography using toluene/hexane as eluent to separate the desired product, followed by recrystallization from a mixed solvent of toluene/ethanol, thereby providing pale yellow solid. This solid was further purified by sublimation to provide 2.5 g of bis (6-(3-diphenylamino)-9-phenyl)carbazolylphenylamine (D).

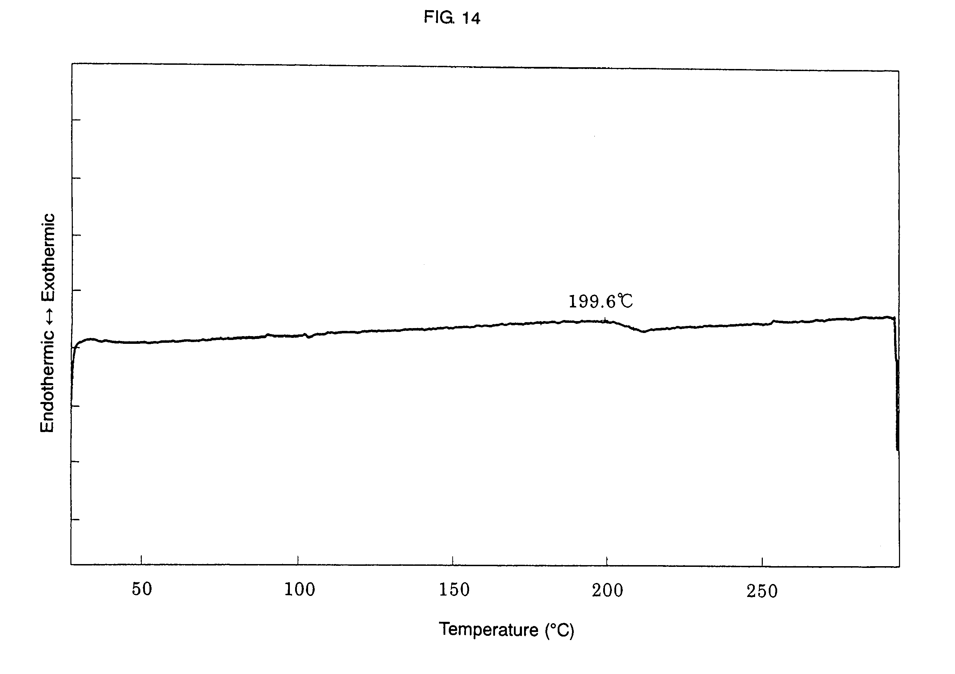

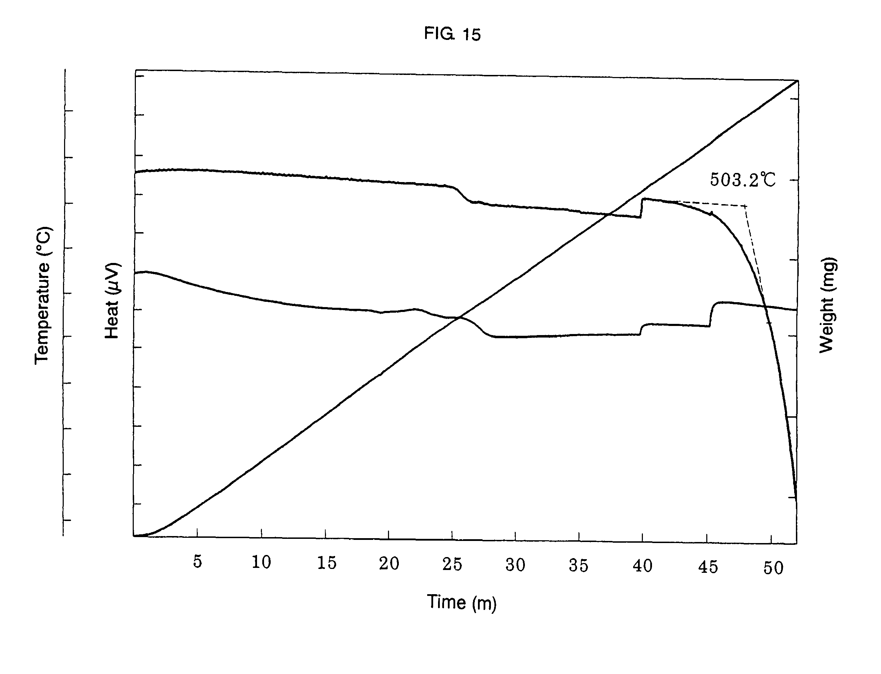

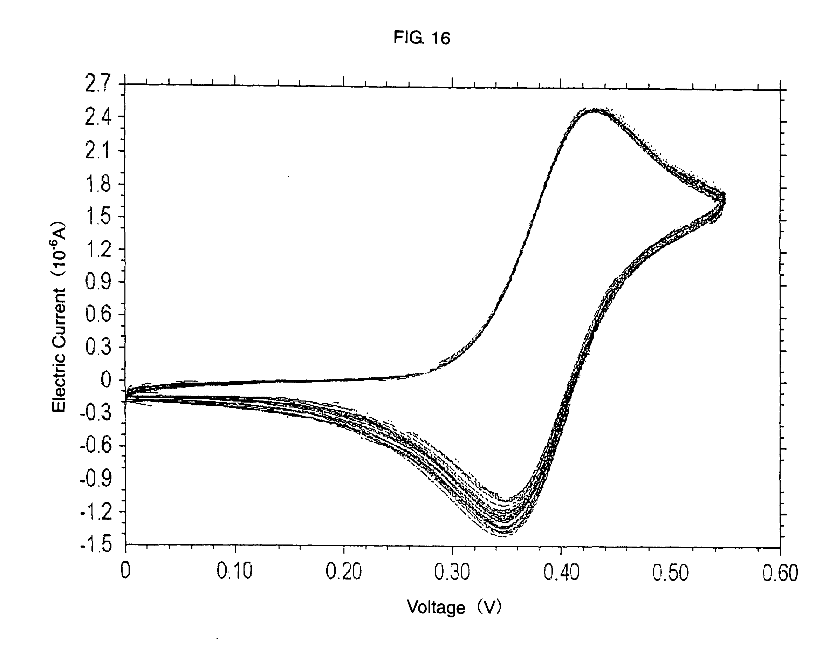

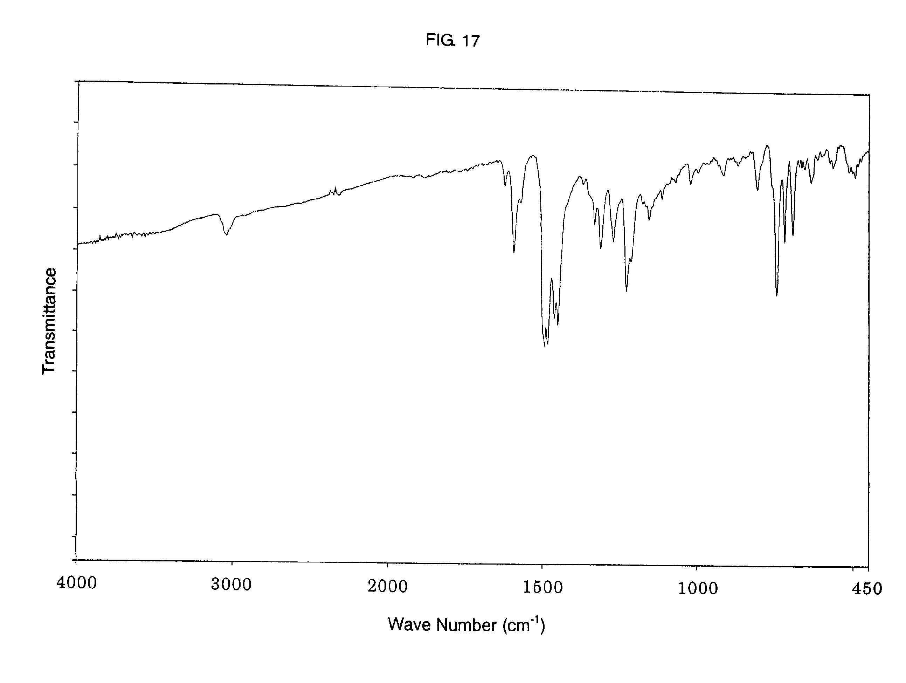

The molecular weight (M⁺ (m/e)) measured by mass analysis and the results of elemental analysis are shown in Table 1. The ionization potential, the glass transition temperature measured by differential scanning calorimetric analysis (DSC), the decomposition temperature measured by thermogravimetric measurement/differential calorimetry (TG/DTA), and the oxidation potential (vs Ag/Ag⁺) measured by cyclic voltammetry (CV) are shown in Table 2. The DSC chart is shown in Fig. 14, the TG/DTA chart is shown in Fig. 15, the CV chart is shown in Fig. 16, and the infrared absorption spectrum (KBr tablet method) is shown in Fig. 17.

### Example 5

3-diphenylamino-6-(9'-(3',6'-bis(diphenylamino)carbazolyl)-9-phenylamine (E) was synthesized according to the following scheme.

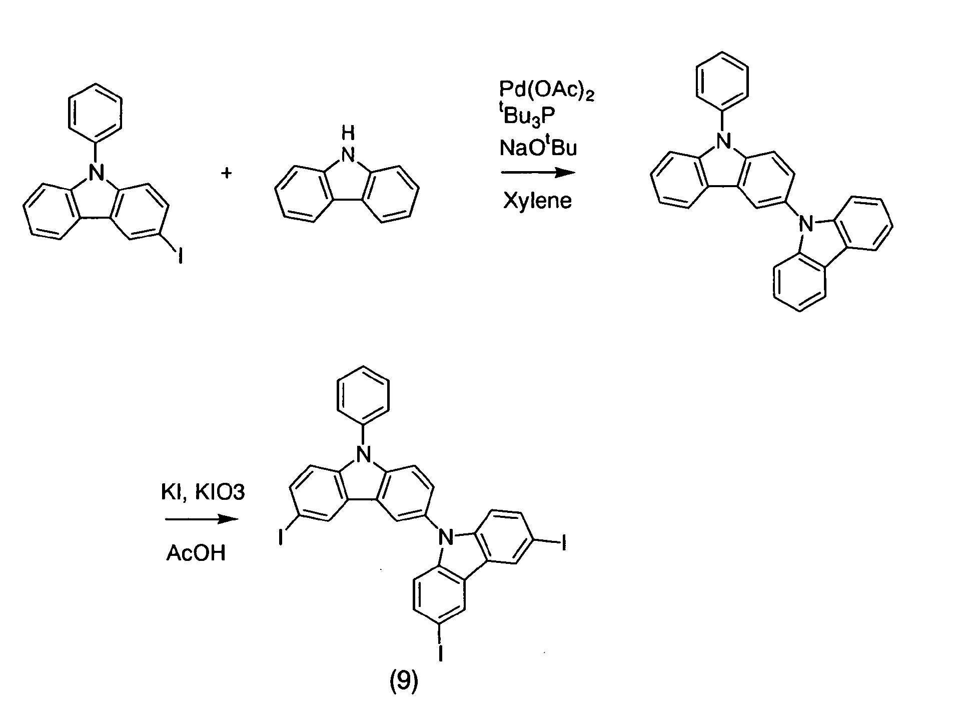

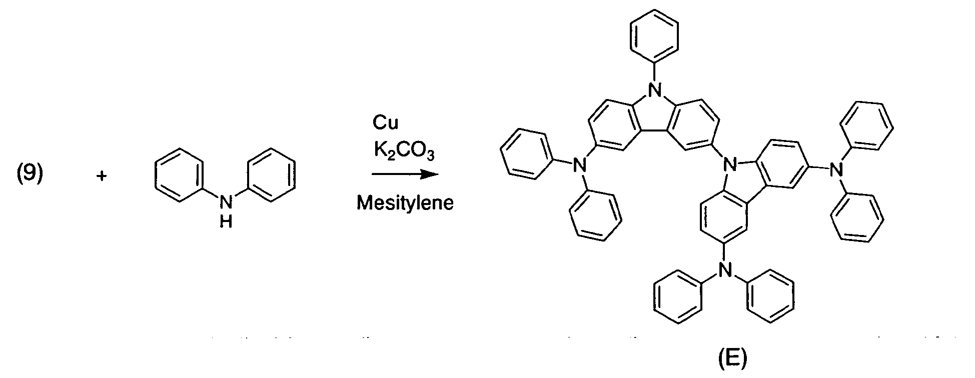

10 g of 3-iodo-9-phenylcarbazole and 5 g of carbazole were reacted in xylene in the presence of 0.006 g of palladium acetate, 0.02g of tri-t-butylphosphine and 3.5 g of sodium t-butoxide. The resulting reaction mixture was extracted with water/toluene, and the resulting organic layer was dropwise added into ethanol to provide 3-(9-carbazolyl)-9-phenylcarbazole as white solid.

The 3-(9-carbazolyl)-9-phenylcarbazole was iodinated with 3.6 g of potassium iodide and 5.5 g of potassium iodate to provide 10.2 g of 3-iodo-6-(9-(3',6'-diiodocarbazolyl)-9-phenylcarbazole (9).

5 g of 3-iodo-6-(9-(3',6'-diiodocarbazolyl)-9-phenylcarbazole (9), 3.8 g of diphenylamine, 3 g of copper and 13 g of potassium carbonate were added to mesitylene, and were reacted at a temperature of 170°C for 15 hours. The resulting reaction mixture was extracted with toluene, and the extract was dropwise added into ethanol to provide yellow solid. The yellow solid was subjected to silica gel column chromatography using a mixed solvent of toluene/hexane as eluent to separate the desired product. The resulting solid was recrystallized, and further purified by sublimation, to provide 2.1 g of 3-diphenylamino-6-(9'-(3',6'-bis(diphenylamino)carbazolyl)-9-phenyl-amine (E) as pale yellow solid.

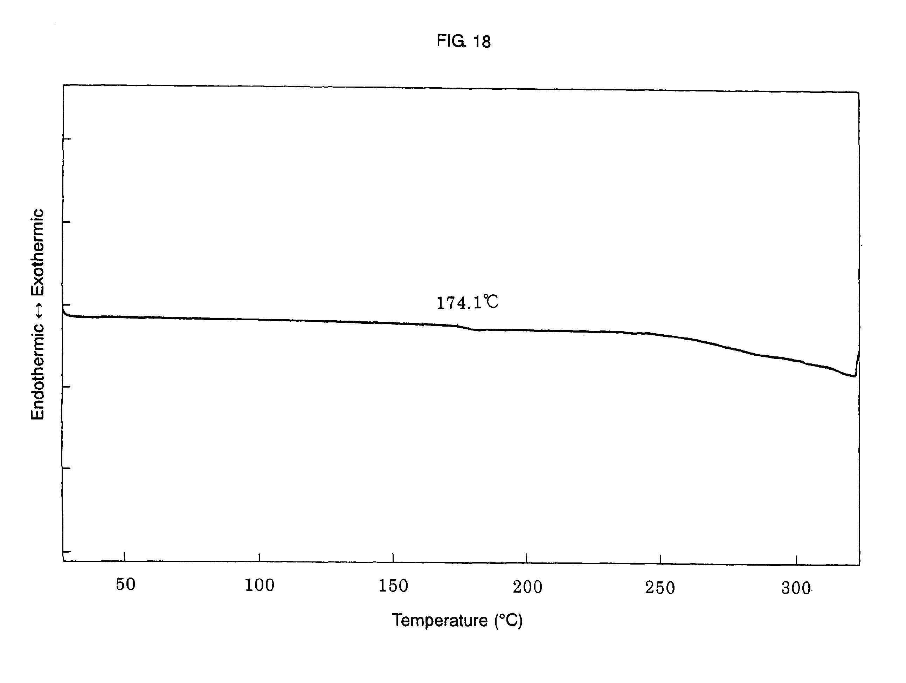

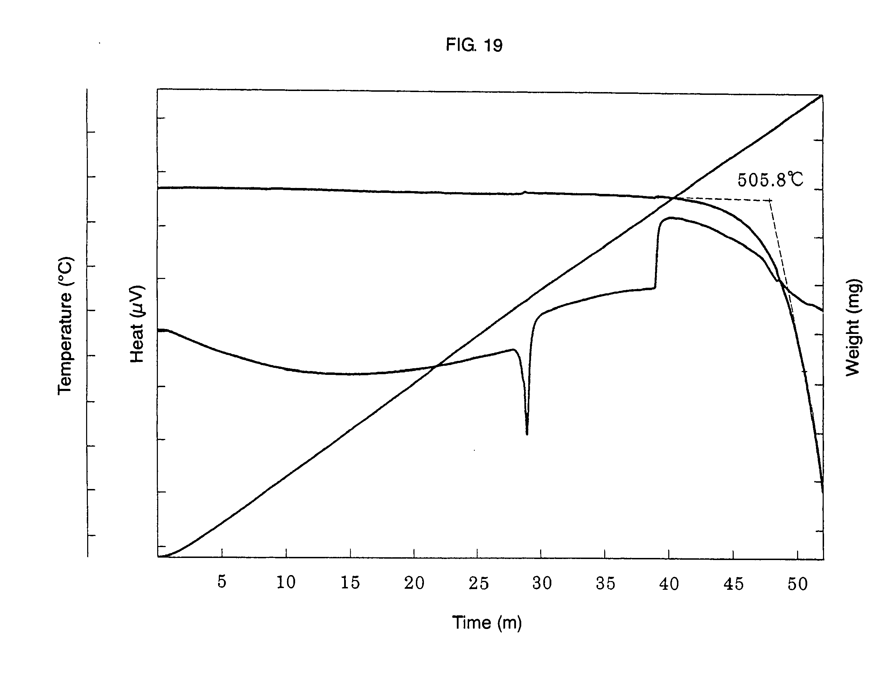

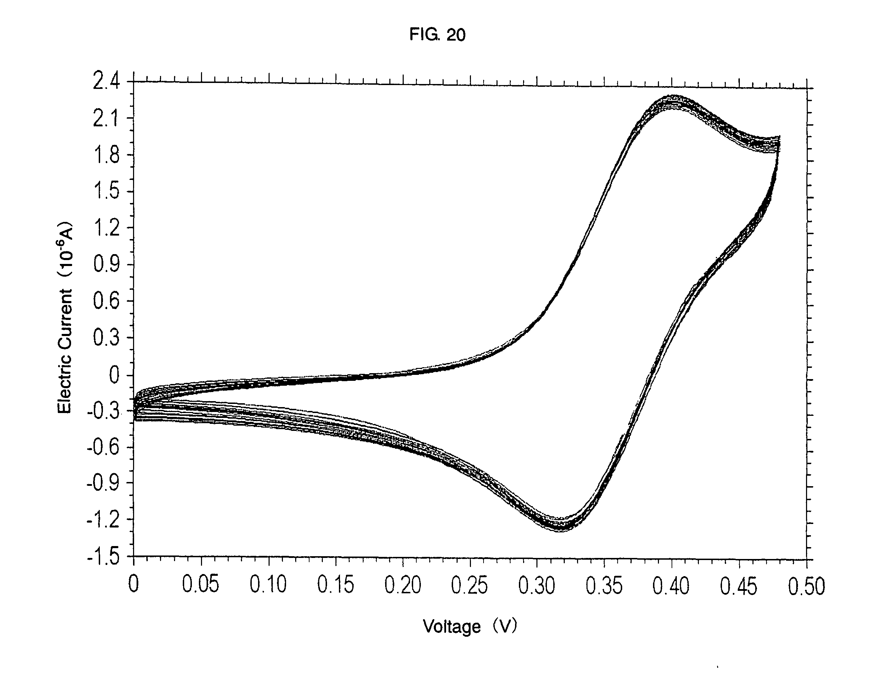

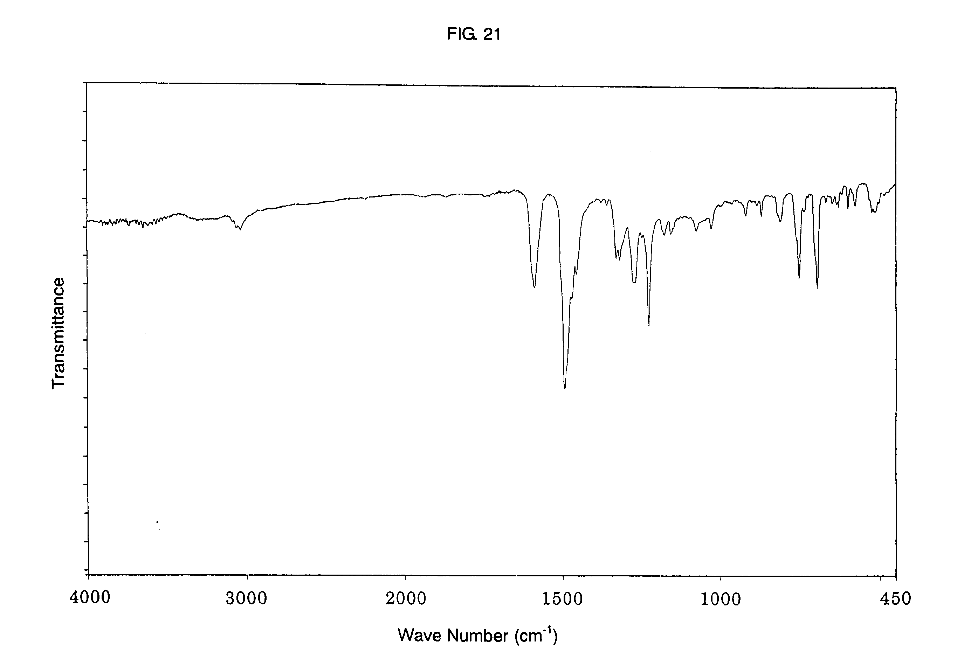

The molecular weight (M⁺ (m/e)) measured by mass analysis and the results of elemental analysis are shown in Table 1. The ionization potential, the glass transition temperature measured by differential scanning calorimetric analysis (DSC), the decomposition temperature measured by thermogravimetric measurement/differential calorimetry (TG/DTA), and the oxidation potential (vs Ag/Ag⁺) measured by cyclic voltammetry (CV) are shown in Table 2. The DSC chart is shown in Fig. 18, the TG/DTA chart is shown in Fig. 19, the CV chart is shown in Fig. 20, and the infrared absorption spectrum (KBr tablet method) is shown in Fig. 21.

### Example 6

Bis(3-(9-(4-diphenylaminophenyl)carbazolyl)phenylamine (F) was synthesized according to the following scheme.

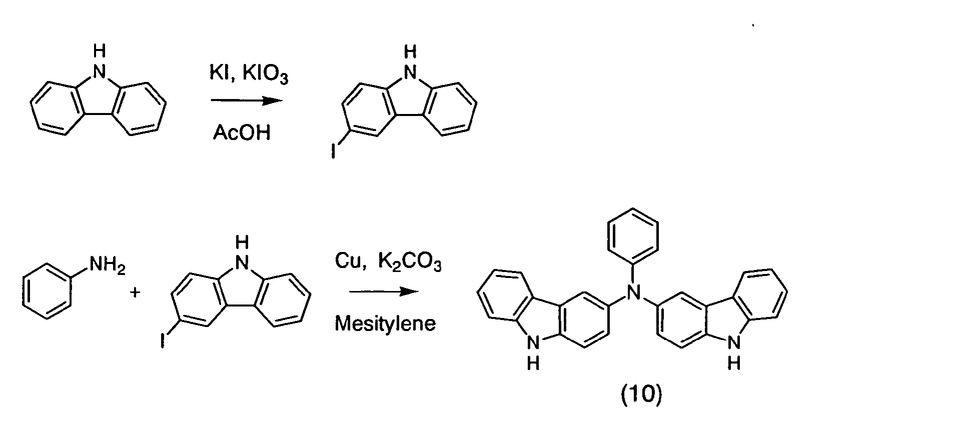

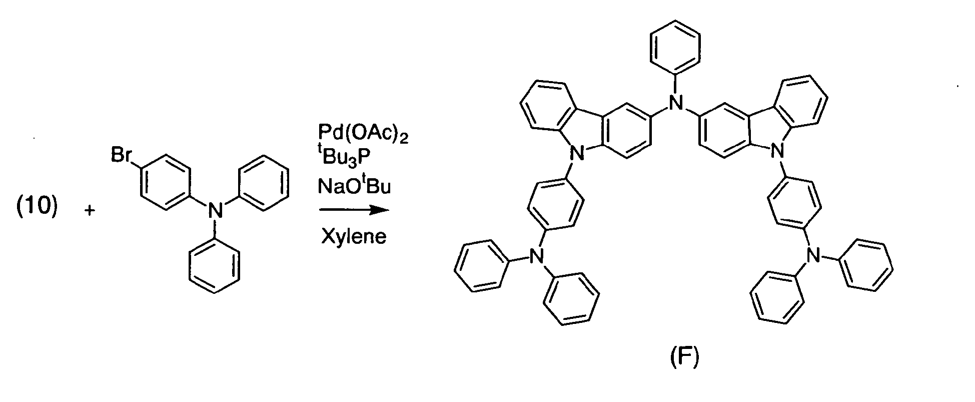

15 g of carbazole, 9.5g of potassium iodide and 6.3 g of potassium iodate were dissolved in acetic acid, and were reacted at a temperature of 50°C for two hours. An aqueous solution of sodium thiosulfate was added to the resulting mixture to reduce iodine therein, and the resulting organic layer was concentrated and subjected to crystallization to provide 17.7 g of 3-iodocarbazole.

15 g of 3-iodocarbazole, 2.1 g of aniline, 35.3 g of potassium carbonate and 7 g of copper powder were added to mesitylene, and were heated with stirring over a period of six hours. After completion of the reaction, the resulting reaction mixture was dissolved in toluene, and the resulting solution was subjected to silica gel column chromatography to separate the desired product, followed by recrystallization from toluene to provide 11.2 g of di-3-carbazolyl-phenylamine (10) as pale yellow solid.

10 g of di-3-carbazolylphenylamine and 18.3 g of 4-bromotriphenylamine were heated in xylene with stirring for three hours in the presence, of 0.005 g of palladium acetate, 0.02g of t-butylphosphine and 3.1 g of sodium t-butoxide. After completion of the reaction, the resulting reaction mixture was washed with toluene/water, and the obtained organic layer was dropwise added into ethanol to provide yellow solid. The solid was collected by filtration, followed by recrystallization and purification by sublimation to provide 1.7 g of bis(3-(9-(4-diphenylaminophenyl)carbazolyl)phenyl-amine (F).

The molecular weight (M⁺ (m/e)) measured by mass analysis and the results of elemental analysis are shown in Table 1. The ionization potential, the glass transition temperature measured by differential scanning calorimetric analysis (DSC), the decomposition temperature measured by thermogravimetric measurement/differential calorimetry (TG/DTA), and the oxidation potential (vs Ag/Ag⁺) measured by cyclic voltammetry (CV) are shown in Table 2.

### Example 7

In place of 4-bromotriphenylamine, 3-iodo-9-phenylcarbazole was reacted with di-3-carbazolylphenylamine in the same manner as in Example 6 to provide bis (3-(9-(3'-(9'-phenylcarbazolyl)))carbazolyl)-phenylamine (G).

The compound was found to have a molecular weight (M⁺ (m/e)) of 906.11 as measured by mass analysis, an ionization potential of 5.14 V, and a glass transition temperature of 180.1°C as measured by differential scanning calorimetric analysis (DSC).

### Example 8

In place of 4-bromotriphenylamine, 9-4-iodophenylcarbazole was reacted with di-3-carbazolylphenylamine in the same manner as in Example 6 to provide bis(3-(9- (4-(9'-carbazolyl)phenyl))carbazolyl)-phenylamine (H).

The compound was found to have a molecular weight (M⁺ (m/e)) of 906.11 as measured by mass analysis, an ionization potential of 5.40 V, and a glass transition temperature of 178.2°C as measured by differential scanning calorimetric analysis (DSC).

### Example 9

N,N,N'-tris(9-phenyl-3-carbazolyl)-N'-phenyl-1,4-diaminobenzene (I) was synthesized according to the following scheme.

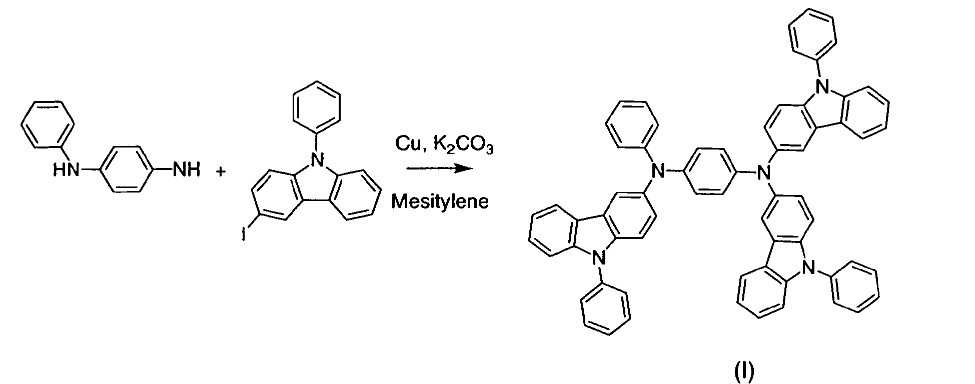

15.6 g of 3-iodo-9-phenylcarbazole, 4.9 g of N-phenyl-1,4-diaminobenzene, 22.4 g of potassium carbonate and 10 g of copper powder were added to mesitylene, and were reacted for 18 hours under reflux. The resulting reaction mixture was dropwise added into ethanol to provide orange solid. The solid was adsorbed onto silica gel and charged in a column, followed by eluting with a mixed solvent of toluene/hexane, thereby a fraction containing the desired product was obtained. The fraction was concentrated and subjected to crystallization from toluene/xylene, followed by purification by sublimation to provide 2.3 g of N,N,N'-tris(9-phenyl-3-carbazolyl)-N'-phenyl-1,4-diaminobenzene (I).

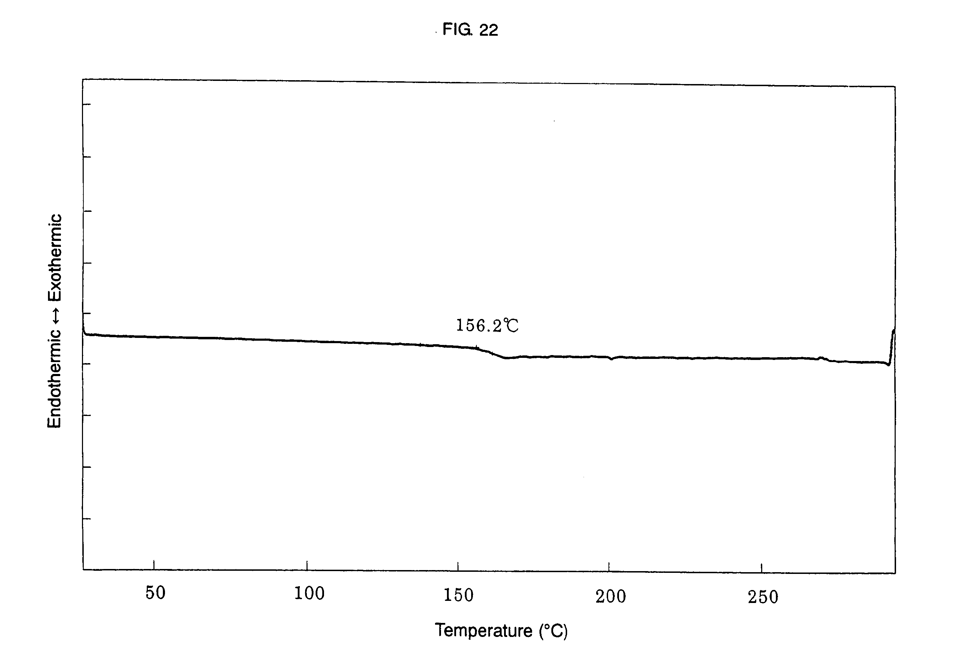

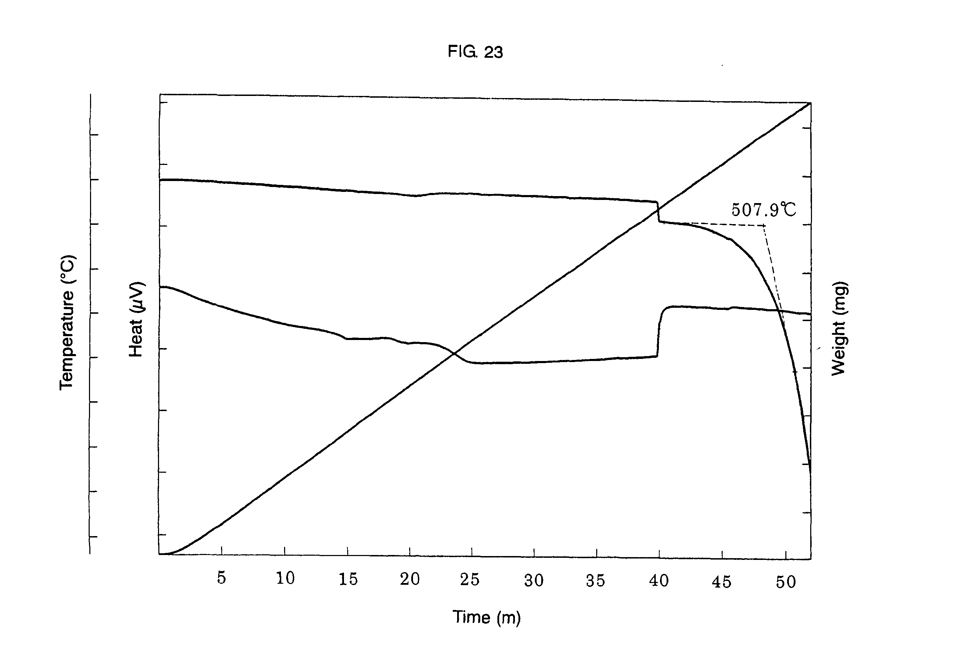

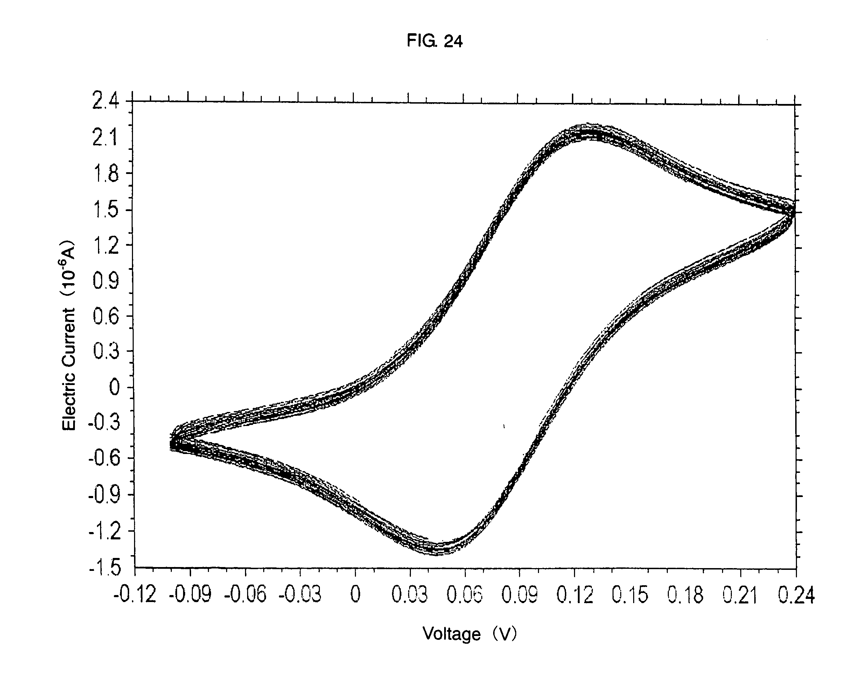

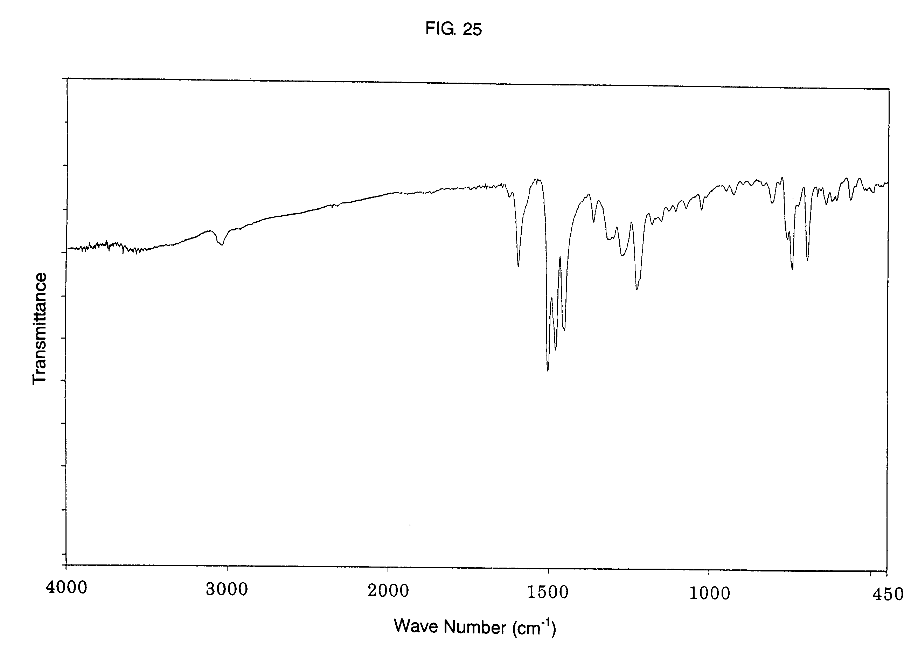

The molecular weight (M⁺ (m/e)) measured by mass analysis and the results of elemental analysis are shown in Table 1. The ionization potential, the glass transition temperature measured by differential scanning calorimetric analysis (DSC), the decomposition temperature measured by thermogravimetric measurement/differential calorimetry (TG/DTA), and the oxidation potential (vs Ag/Ag⁺) measured by cyclic voltammetry (CV) are shown in Table 2. The DSC chart is shown in Fig. 22, the TG/DTA chart is shown in Fig. 23, the CV chart is shown in Fig. 24, and the infrared absorption spectrum (KBr tablet method) is shown in Fig. 25.

### Example 10

N-phenyl-N'-(4-(9'-carbazolyl)phenyl)-N,N'-bis(9"-phenyl-3"-carbazolyl)-1,4-diaminobenzene (J) was synthesized according to the following scheme.

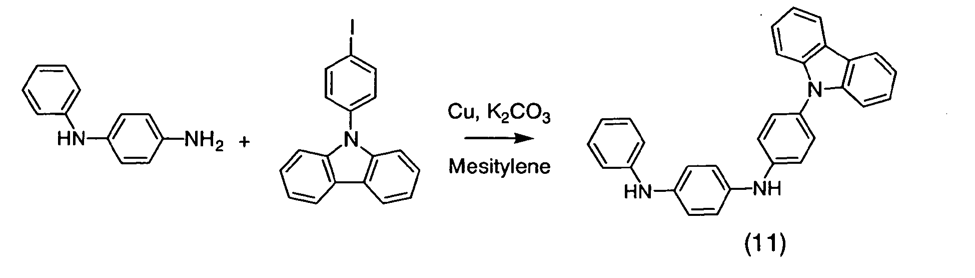

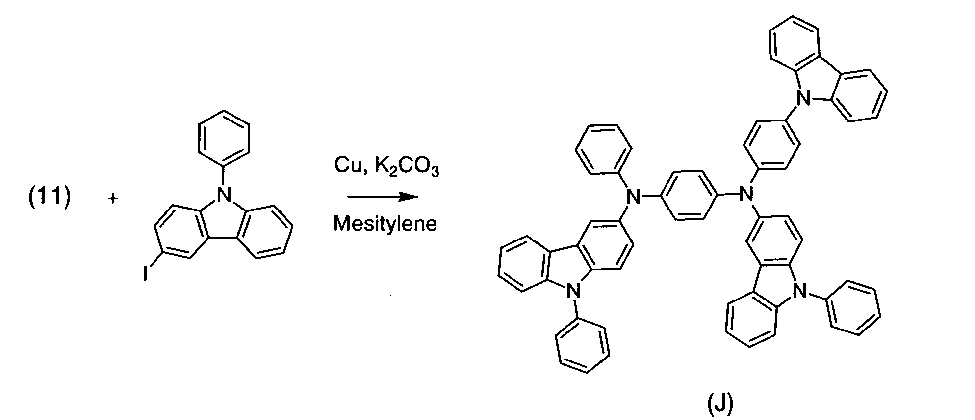

5 g of 9-(4-iodophenyl)carbazole, 4.9 g of N-phenyl-1,4-diaminobenzene, 9.3 g of potassium carbonate and 5 g of copper powder were added to mesitylene, and were reacted under reflux for 5 hours. The resulting solution was subjected to silica gel chromatography to separate the desired product, followed by recrystallization, to provide N-phenyl-N'-(4-(9-carbazolyl)phenyl-1,4-diaminobenzene (11) as pale yellow solid.

The solid was reacted with 5.9 g of 3-iodo-9-phenylcarbazole so that 1.9 g of N-phenyl-N,N'-bis(3-(9-phenylcarbazolyl)phenyl)-N'-(4-(9'-carbazolyl)phenyl-1,4-diaminobenzene (J) was obtained.

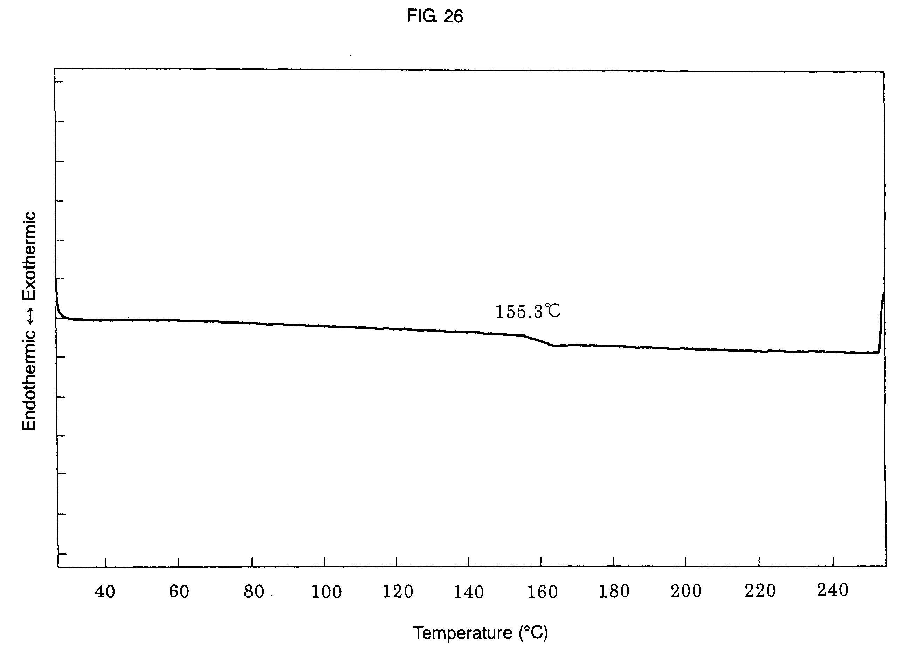

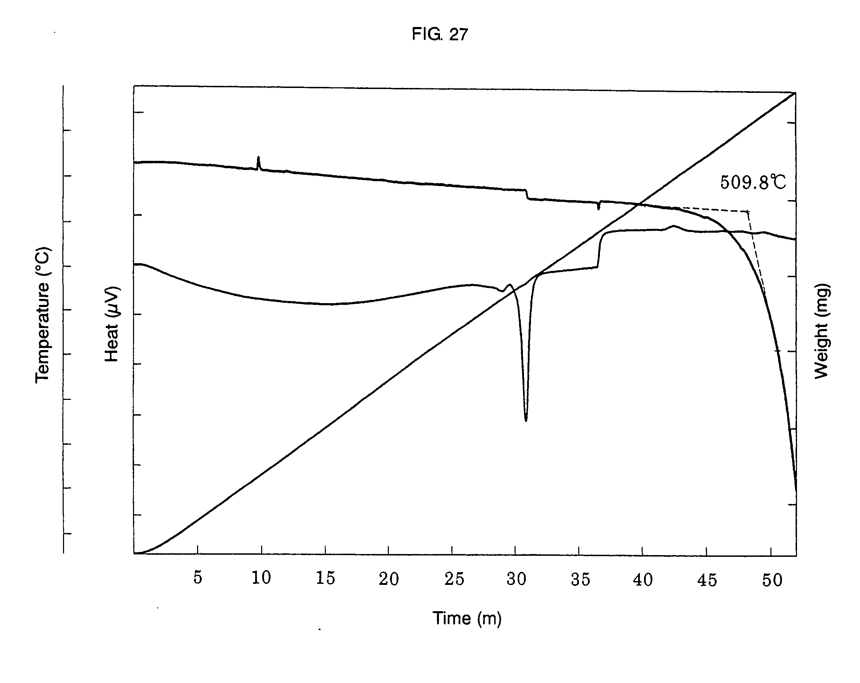

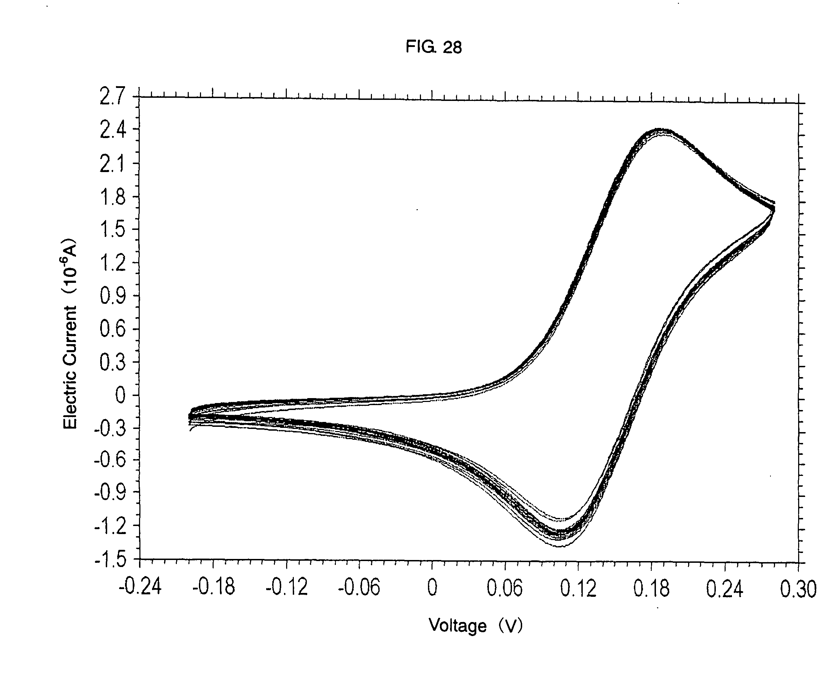

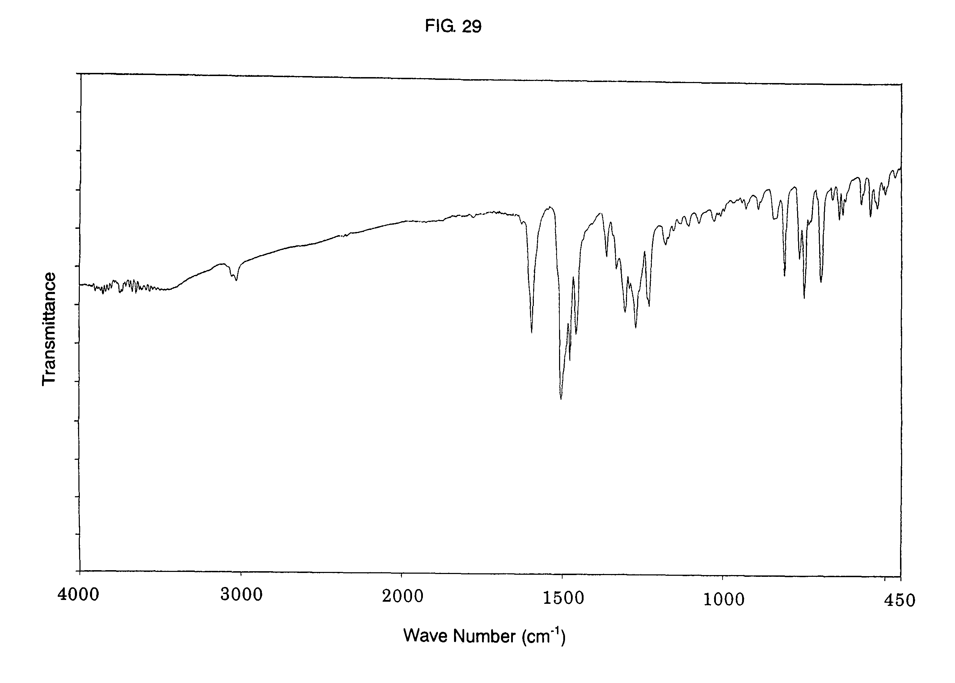

The molecular weight (M⁺ (m/e)) measured by mass analysis and the results of elemental analysis are shown in Table 1. The ionization potential, the glass transition temperature measured by differential scanning calorimetric analysis (DSC), the decomposition temperature measured by thermogravimetric measurement/differential calorimetry (TG/DTA), and the oxidation potential (vs Ag/Ag⁺) measured by cyclic voltammetry (CV) are shown in Table 2. The DSC chart is shown in Fig. 26, the TG/DTA chart is shown in Fig. 27, the CV chart is shown in Fig. 28, and the infrared absorption spectrum (KBr tablet method) is shown in Fig. 29.

### Example 11

N,N'-diphenyl-N,N'-bis(4-(9-phenylcarbazolyl)phenyl) -1,4-diaminobenzene (K) was synthesized according to the following scheme.

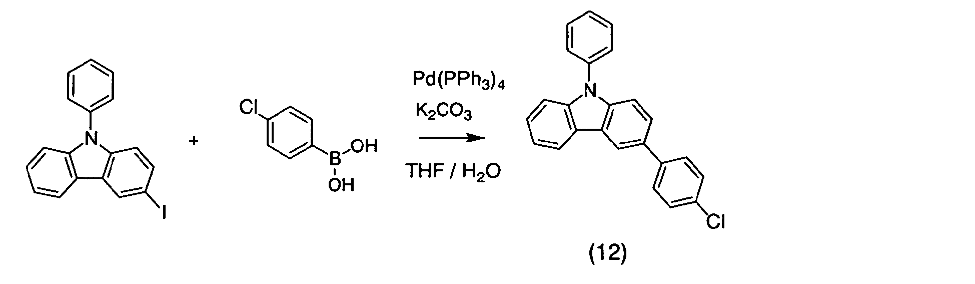

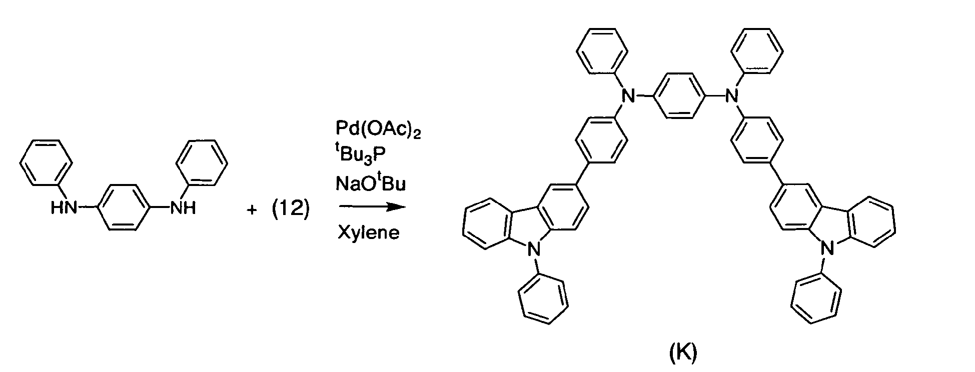

5 g of p-chlorophenylboric acid and 7.9 g of 3-iodo-9-phenylcarbazole were subjected to Suzuki coupling reaction in a mixed solvent of tetrahydrofuran/water in the presence of 0.05 g of tetrakistriphenylphosphine palladium and 8.8 g of potassium carbonate. After completion of the reaction, the resulting reaction mixture was extracted with toluene to obtain organic material. The organic material was crystallized from xylene twice, thereby providing 3-(4-chlorophenyl)-9-phenylcarbazole (12).

6.3 g of 3-(4-chlorophenyl)-9-phenylcarbazole was reacted with 2.1 g of N,N'-diphenyl-4,4-diaminobenzene in xylene in the presence of 0.005 g of palladium acetate, 0.018g of tri-t-butylphosphine and 1.9 g of sodium t-butoxide. After completion of the reaction, the resulting reaction mixture was subjected to crystallization from toluene/ethanol to obtain the desired product, which was then washed and purified by sublimation thereby providing 3.1 g of N,N'-diphenyl-N,N'-bis-(4-(9-phenylcarbazolyl)-phenyl)-1,4-diaminobenzene (K) as white solid.

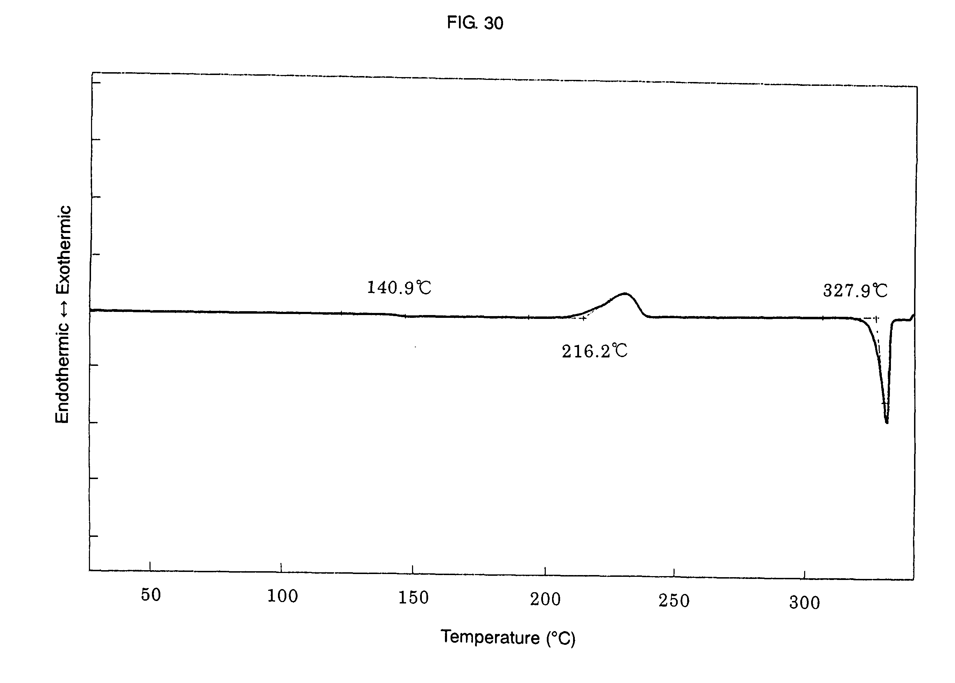

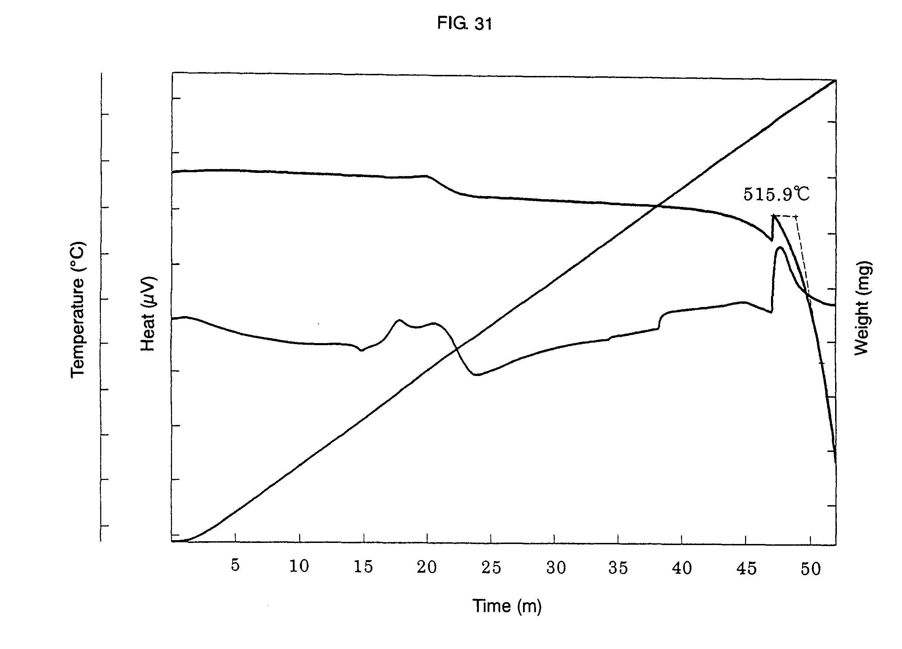

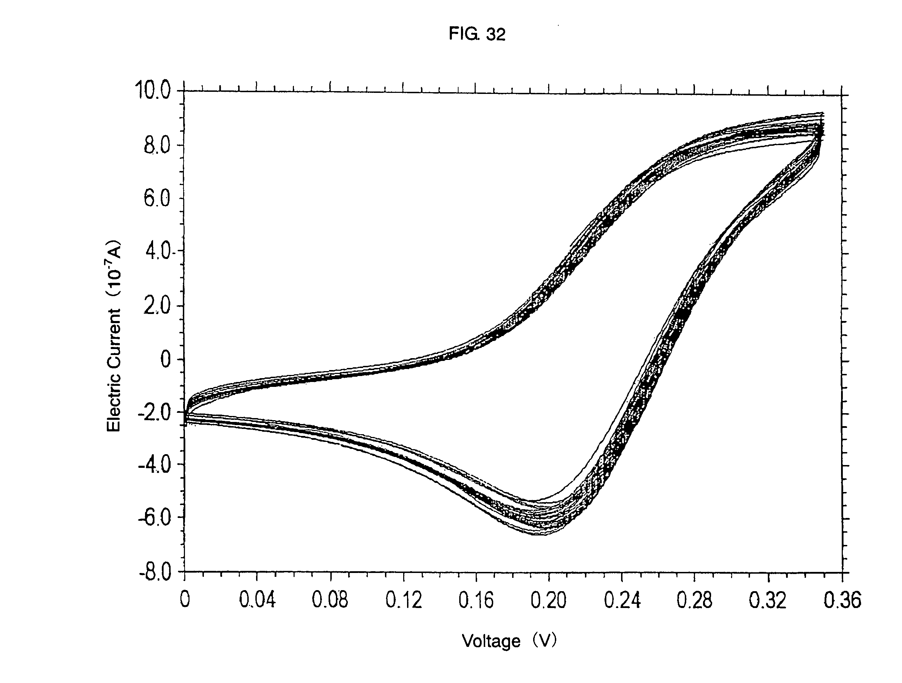

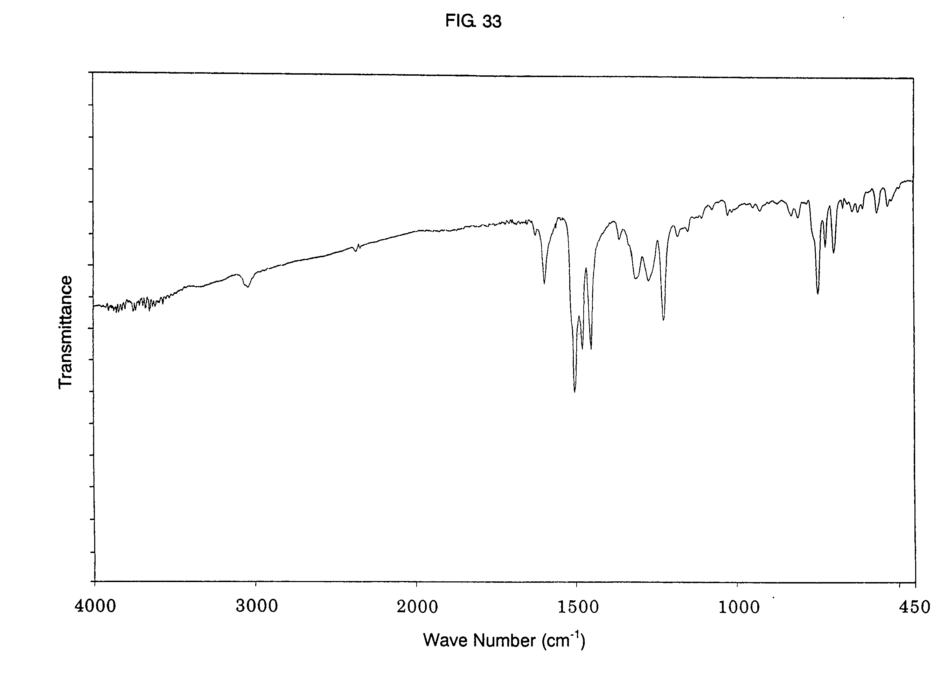

The molecular weight (M⁺ (m/e)) measured by mass analysis and the results of elemental analysis are shown in Table 1. The ionization potential, the glass transition temperature measured by differential scanning calorimetric analysis (DSC), the decomposition temperature measured by thermogravimetric measurement/differential calorimetry (TG/DTA), and the oxidation potential (vs Ag/Ag⁺) measured by cyclic voltammetry (CV) are shown in Table 2. The DSC chart is shown in Fig. 30, the TG/DTA chart is shown in Fig. 31, the CV chart is shown in Fig. 32, and the infrared absorption spectrum (KBr tablet method) is shown in Fig. 33.

### Example 12

3-(9-phenylcarbazolyl)-3'-(6'-diphenylamino-9'-phenyl)-carbazolylphenylamine (L) was synthesized according to the following scheme.

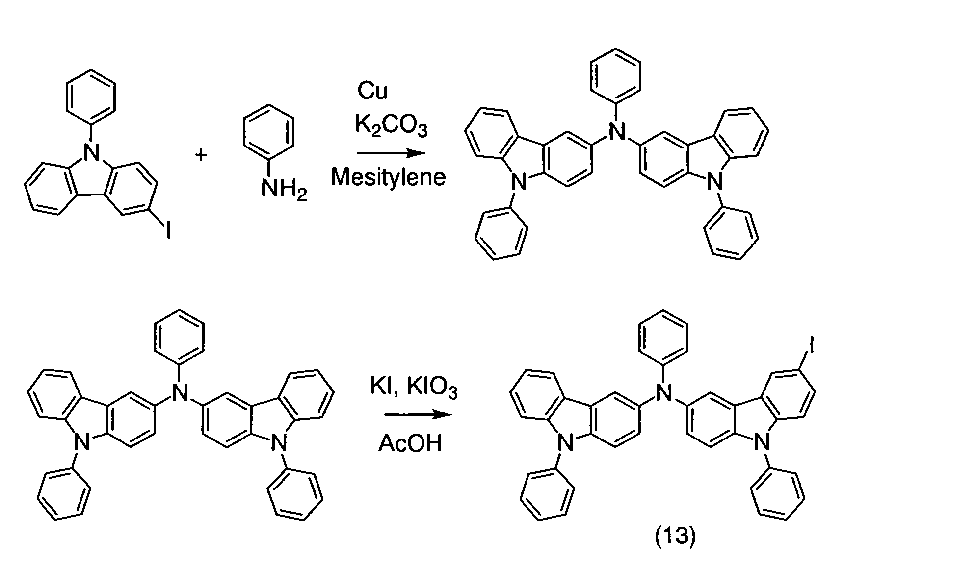

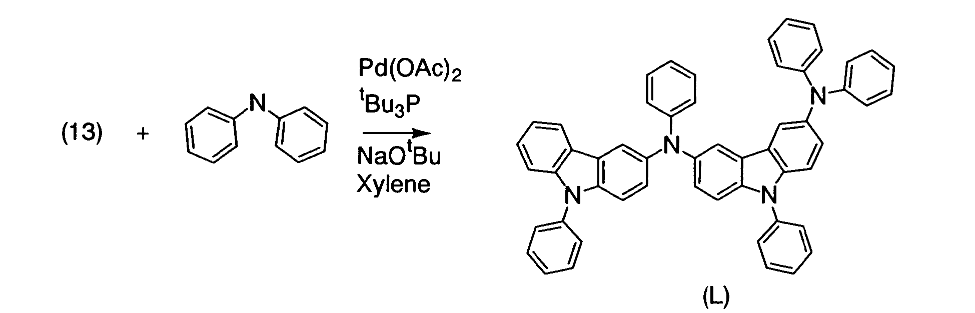

5.5 g of 3-iodo-9-phenylcarbazole and 0.7 g of aniline were reacted at 160°C for 3 hours in the presence of 3 g of copper powder and 10.5 g of potassium carbonate in mesitylene. Toluene was added to the resulting reaction mixture, and the resultant mixture was heated with stirring to dissolve the reaction product. The resulting solution was filtered to remove solid components therefrom. The solution was concentrated, and the obtained concentrate was dissolved in acetic acid. To the resulting solution were added 0.4 g of potassium iodide and 0.5 g of potassium iodate to carry out an iodination reaction. The resulting reaction mixture was neutralized with an aqueous solution of sodium thiosulfate, followed by separation and purification by using an alumina column, thereby 2.1 g of 3-(9-phenylcarbazolyl)-3'-(6'-iodo-9'-phenyl)carbazolylphenylamine (13) was obtained as pale yellow solid.

2.0 g of 3-(9-phenylcarbazolyl)-3'-(6'-iodo-9'-phenyl)carbazolyl-phenylamine (13) was reacted with 0.9 g of diphenylamine at a temperature of 120°C in xylene for six hours in the presence of 0.008 g of palladium acetate, 0.028 g of tri-t-butylphosphine and 0.55 g of sodium t-butoxide. The resulting reaction mixture was extracted with water/toluene, and the obtained organic layer was concentrated. The concentrated solution was dropwise added into ethanol, and the crystallized yellow solid was collected by filtration and then dissolved in toluene. The thus obtained solution was subjected to silica gel column chromatography to separate and purify the desired product, followed by further purification by sublimation to provide 0.9 g of 3-(9-phenylcarbazolyl)-3'-(6'-diphenylamino)-(9'-phenylcarbazolyl)-phenylamine (L) as pale yellow solid.

The compound was found to have a molecular weight (M⁺ (m/e)) of 742.93 as measured by mass analysis, an ionization potential of 5.21 V, and a glass transition temperature of 124.9°C as measured by differential scanning calorimetric analysis (DSC).

### Example 13

3-(9-phenylcarbazolyl)-3'-(6'-(9"-carbazolyl)-9'-phenyl)-carbazolyl)phenylamine (M) was synthesized according to the following scheme.

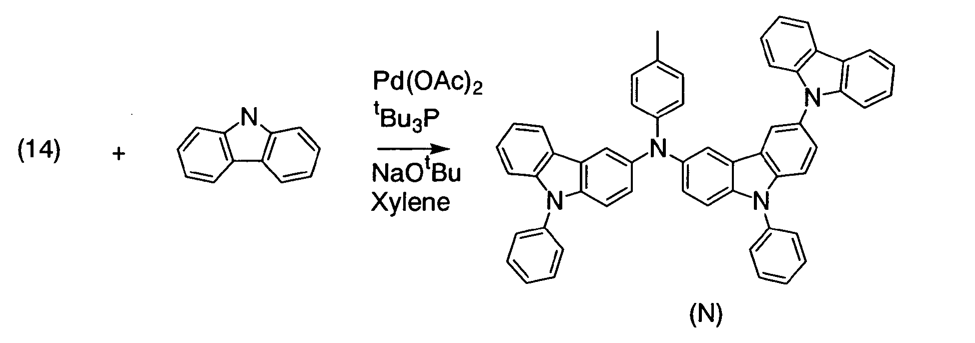

2.0 g of 3-(9-phenylcarbazolyl)-3'-(6'-iodo-9'-phenyl)carbazolyl-phenylamine (13) prepared in Example 12 and 1.0 g of carbazole were reacted in xylene in the presence of 0.008 g of palladium acetate, 0.028 g of tri-t-butylphosphine and 0.55 g of sodium t-butoxide at a temperature of 120°C for six hours. The resulting reaction mixture was extracted with water/toluene and the obtained organic layer was concentrated. The concentrated solution was dropwise added into ethanol, and the resultant crystallized yellow solid was collected by filtration. The solid was then dissolved in toluene, and the resulting solution was subjected to silica gel column chromatography to separate and purify the desired product, followed by further purification by sublimation, thereby providing 1.2 g of 3-(9-phenylcarbazolyl)-3'-(6'-(9"-carbazolyl)-9'-phenyl)carbazolyl)phenylamine (M) as pale yellow solid.

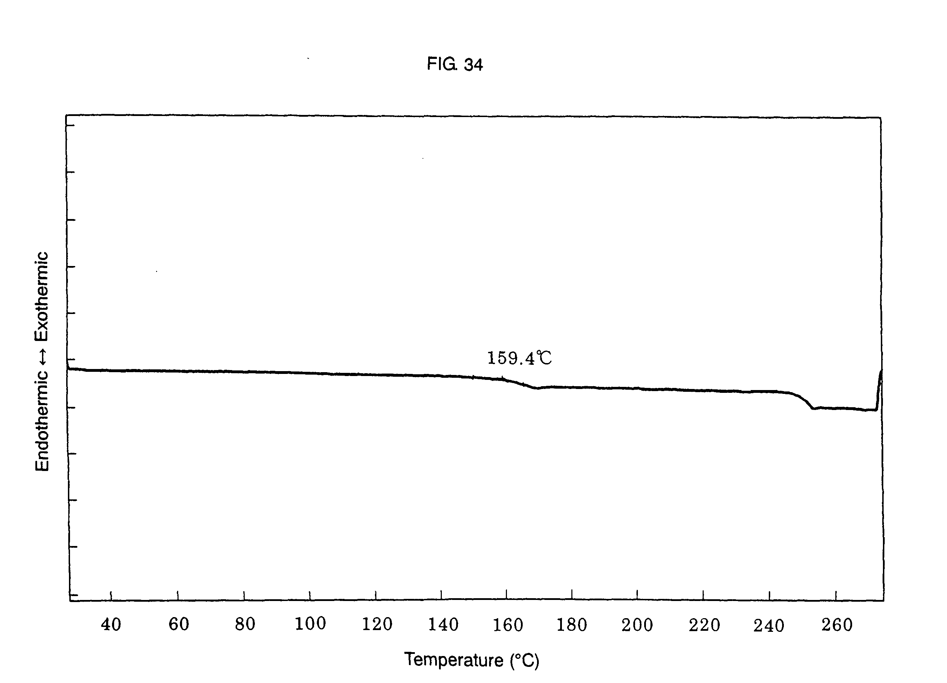

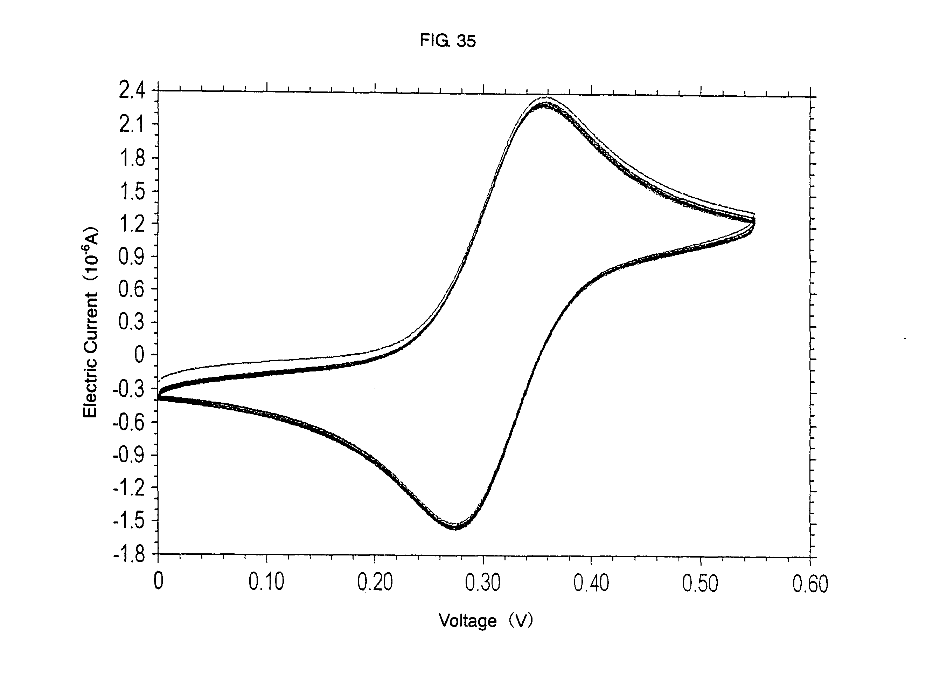

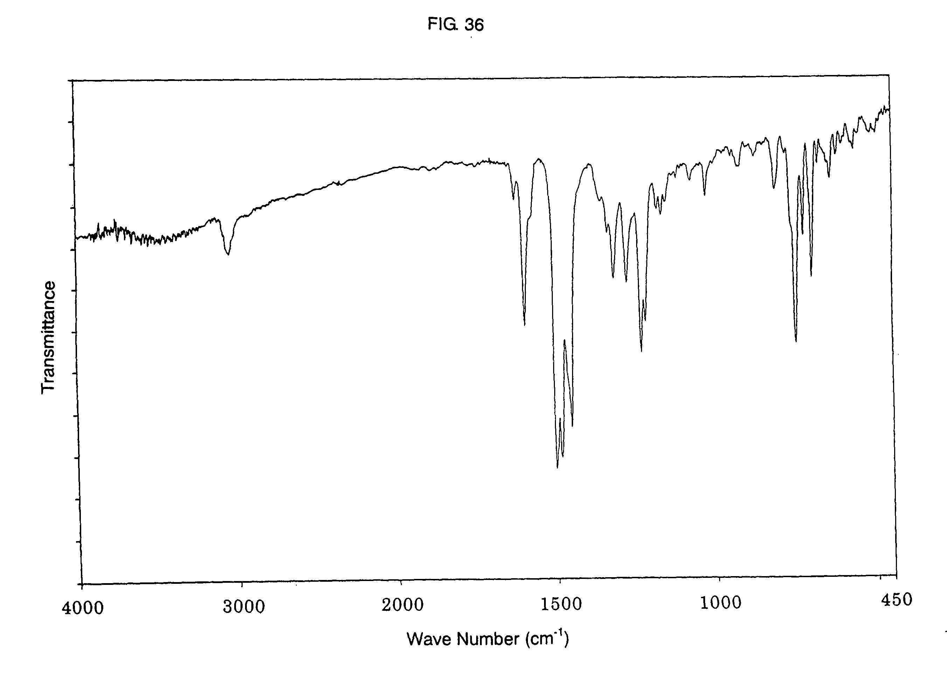

The molecular weight (M⁺ (m/e)) measured by mass analysis and the results of elemental analysis are shown in Table 1. The ionization potential, the glass transition temperature measured by differential scanning calorimetric analysis (DSC), the decomposition temperature measured by thermogravimetric measurement/differential calorimetry (TG/DTA), and the oxidation potential (vs Ag/Ag⁺) measured by cyclic voltammetry (CV) are also shown in Table 2. The DSC chart is shown in Fig. 34, the CV chart is shown in Fig. 35, and the infrared absorption spectrum (KBr tablet method) is shown in Fig. 36.

### Example 14

3-carbazolyl-3'-(6'-(9"-carbazolyl)-9'-phenylcarbazolyl)-p-methylphenylamine (N) was synthesized according to the following scheme.

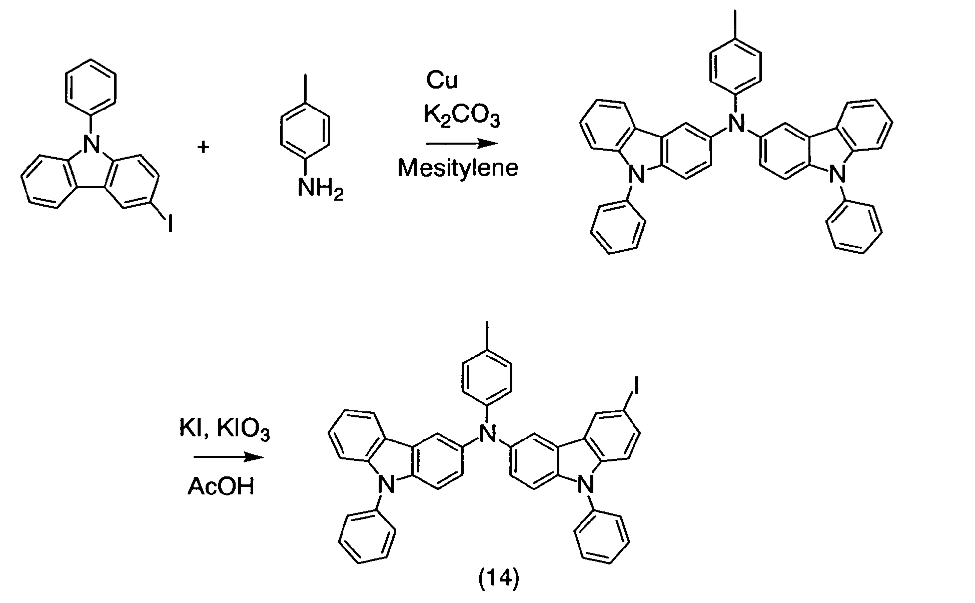

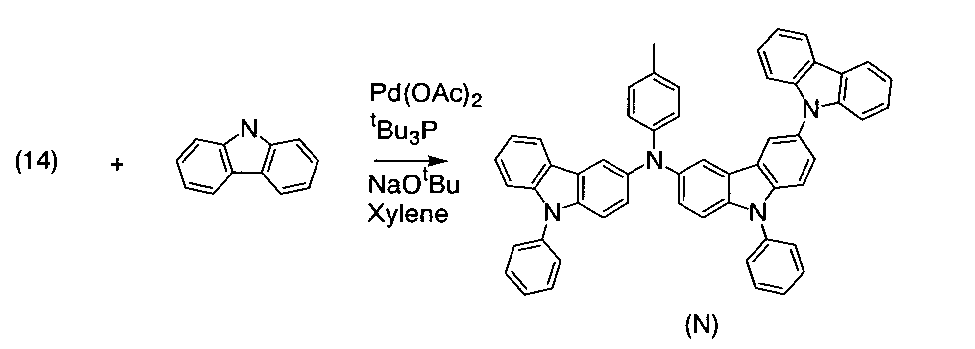

In place of aniline, p-toluidine was reacted with 3-iodo-9-phenylcarbazole otherwise in the same manner as in Example 12. The resulting reaction product was iodinated in the same manner to provide 3-(9-phenyl-3-carbazolyl)-3'-(6'-iodo-9'-phenylcarbazolyl)-4-methylphenylamine (14), which was then reacted with carbazole in the same manner, thereby 3-carbazolyl-3'-(6'-(9"-carbazolyl)-9'-phenylcarbazolyl)-p-methylphenyl amine (N) was obtained.

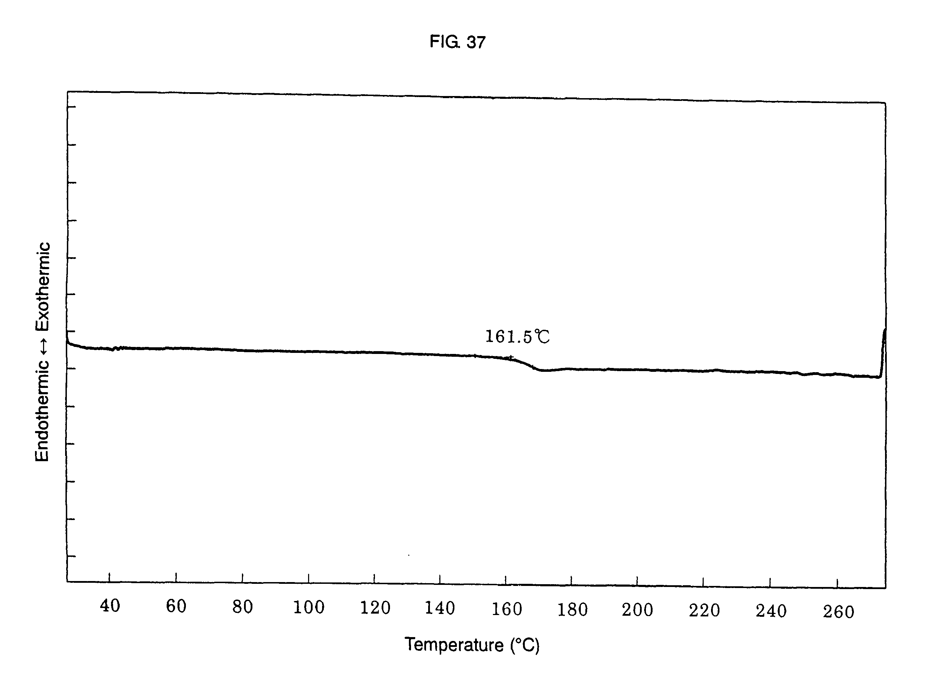

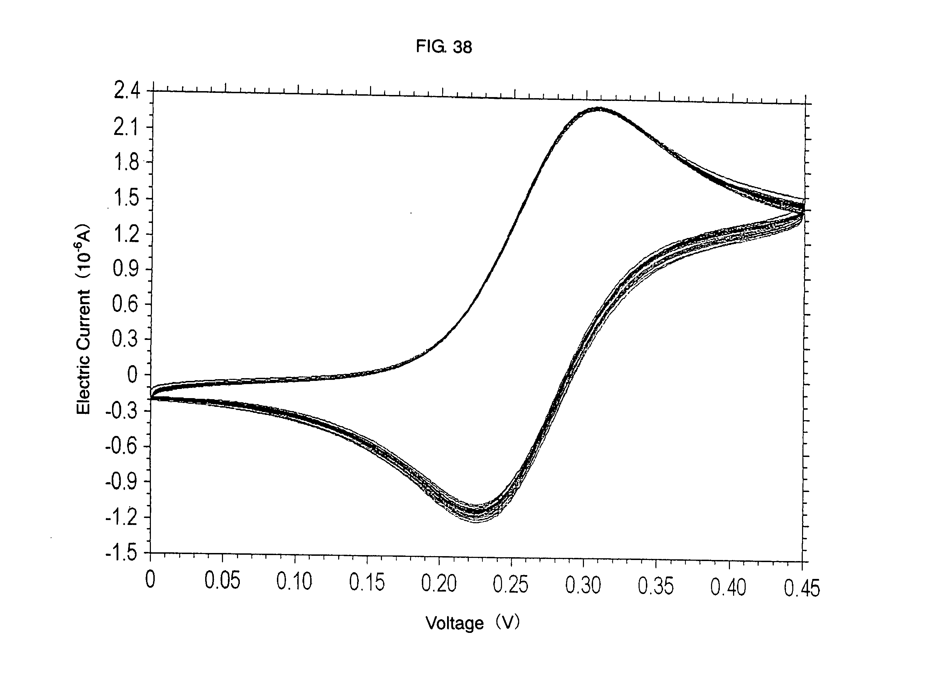

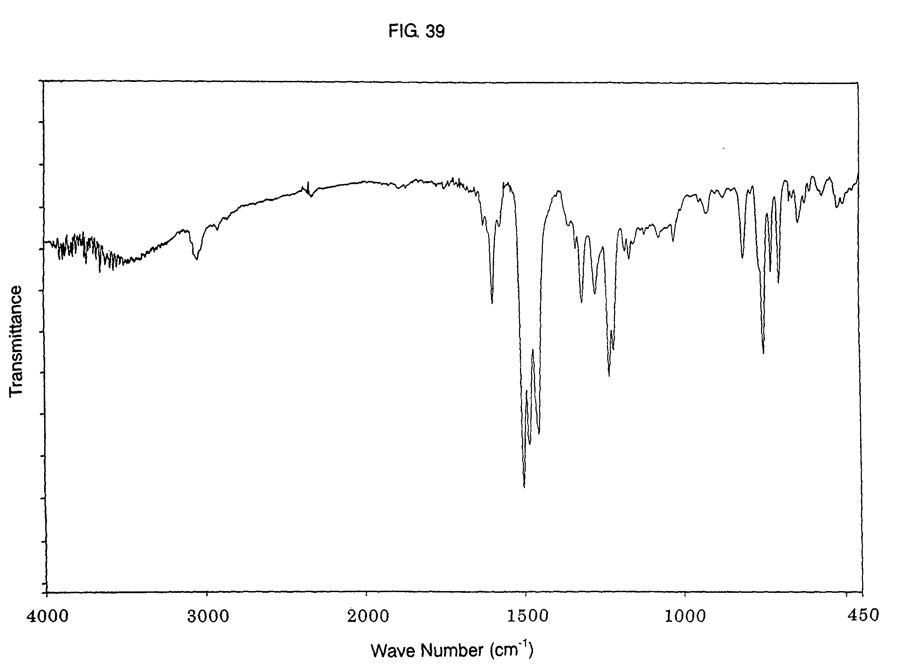

The molecular weight (M+ (m/e)) measured by mass analysis and the results of elemental analysis are shown in Table 1. The ionization potential, the glass transition temperature measured by differential scanning calorimetric analysis (DSC), the decomposition temperature measured by thermogravimetric measurement/differential calorimetry (TG/DTA), and the oxidation potential (vs Ag/Ag⁺) measured by cyclic voltammetry (CV) are also shown in Table 2. The DSC chart is shown in Fig. 37, the CV chart is shown in Fig. 38, and the infrared absorption spectrum (KBr tablet method) is shown in Fig. 39.

**TABLE 1**

| Example I | Compound | Molecular Weight | Elemental Analysis (% by weight) | | |
|---|---|---|---|---|---|
| | | | C | H | N |
| Example 1 | A | 910.14 | 86.91 (87.08) | 5.31 (5.34) | 7.78 (7.58) |
| Example 2 | B | 910.14 | 87.07 (87.08) | 5.34 (5.34) | 7.59 (7.58) |
| Example 3 | C | 906.11 | 87.49 (87.49) | 4.79 (4.78) | 7.72 (7.73) |
| Example 4 | D | 910.14 | 87.15 (87.10) | 5.21 (5.21) | 7.64 (7.70) |
| Example 5 | E | 910.14 | 87.11 (87.10) | 5.20 (5.21) | 7.70 (7.70) |
| Example 6 | F | 910.14 | 87.09 (87.10) | 5.20 (5.21) | 7.71 (7.70) |
| Example 9 | I | 908.13 | 87.27 (87.29) | 4.99 (4.99) | 7.74 (7.71) |
| Example 10 | J | 908.13 | 87.32 (87.29) | 4.98 (4.99) | 7.70 (7.71) |
| Example 11 | K | 895.13 | 86.91 (85.56) | 5.19 (5.18) | 6.25 (6.26) |
| Example 13 | M | 740.92 | 87.33 (87.30) | 5.15 (5.16) | 7.51 (7.54) |
| Example 14 | N | 754.92 | 87.56 (87.54) | 4.90 (4.90) | 7.54 (7.56) |

| | | | | | |
|---|---|---|---|---|---|
| (Notes) The values in parenthesis are theoretical values. | | | | | |

**TABLE 2**

| Example I | Compound | Ionization Potential | Tg | Td | Oxidation Potential |
|---|---|---|---|---|---|
| | | (eV) | (°C) | (°C) | (eV) |
| Example 1 | A | 5.02 | 136.4 | 508.4 | 0.037 |
| Example 2 | B | 5.02 | 135.0 | 508.2 | 0.201 |
| Example 3 | C | 5.34 | 199.6 | 503.2 | 0.388 |
| Example 4 | D | 5.19 | 168.2 | 504.3 | 0.305 |
| Example 5 | E | 5.30 | 174.1 | 505.8 | 0.355 |
| Example 6 | F | 5.22 | 137 | 502.8 | 0.237 |
| Example 9 | I | 4.93 | 156.2 | 507.9 | 0.090 |
| Example 10 | J | 5.03 | 155.3 | 509.8 | 0.149 |
| Example 11 | K | 5.09 | 140.9 | 515.9 | 0.253 |
| Example 13 | M | 5.30 | 159.4 | 501.7 | 0.315 |
| Example 14 | N | 5.23 | 161.5 | 509.0 | 0.266 |

### Example II (Performance of Organic Electroluminescence Elements)

### Example I

A sheet of plate glass having an ITO coating on one face (available from Sanyo Vacuum K.K.) was subjected to ultrasonic cleaning using acetone and then steam cleaning using methanol, followed by irradiation with ultraviolet rays by using a low-pressure mercury lamp for 10 minutes. Immediately after the irradiation, the carbazole derivative (A) of the invention was vacuum evaporated to form a hole injecting layer 50 nm thick and then α-NPD was vacuum evaporated on the hole injecting layer to form a hole transporting layer 10 nm thick in this order on the ITO coating by using a vacuum evaporation apparatus. Subsequently, an emission layer 75 nm thick was formed of tris(8-quinolinol)aluminum (Alq₃) on the hole transporting layer, and then a lithium fluoride layer 0.5 nm thick and an aluminum layer 100 nm thick were layered in this order on the emission layer to form a cathode, thereby an organic electroluminescence element was obtained.

The current efficiency of luminance, the energy efficiency of emission, the luminance half-life period as defined by driving time until the luminance came to half the initial luminance of 1000 cd/m² and the maximum luminance when the organic electroluminescence element was driven at a current density of 25 mA/cm² are shown in Table 3.

### Examples 2-9

In place of carbazole derivative (A), the compounds shown in Table 1 were used, and otherwise in the same manner as in Example 1, organic electroluminescence elements were prepared, respectively. The performance of each of the organic electroluminescence elements was evaluated in the same manner. The results are shown in Table 3.

### Examples 10-13

In place of carbazole derivative (A), the carbazole derivatives shown in Table 1 were used to prepare hole injecting layers 50 nm thick, and in place of α-NPD, the compounds shown in Table 1 were used to prepare hole transporting layers 10 nm thick, and otherwise in the same manner as in Example 1, organic electroluminescence elements were prepared, respectively. The performance of each of the organic electroluminescence elements was evaluated in the same manner. The results are shown in Table 3.

### Comparative Example 1

In place of carbazole derivative (A), CuPC was used to prepare a hole injecting layer, and otherwise in the same manner as in Example 1, an organic electroluminescence element was prepared, and the performance was evaluated in the same manner as in Example 1. The results are shown in Table 3.

**TABLE 3**

| Example II | Hole Injecting Agent | Hole Trasporting Agent | Current Efficiency of Luminance (cd/A) | Energy Efficiency of Emission (lm/W) | Luminance Half-Period (h) | Maximum Luminance (cd/m²) |
|---|---|---|---|---|---|---|
| | Compound | Compound | | | | |
| Example 1 | A | α-NPD | 5.47 | 4.60 | 12553 | 32945 |
| Example 2 | B | α-NPD | 3.69 | 2.82 | 11872 | 31829 |
| Example 3 | C | α-NPD | 3.51 | 2.66 | 13590 | 34139 |
| Example 4 | D | α-NPD | 4.38 | 2.92 | 9287 | 30026 |
| Example 5 | E | α-NPD | 4.95 | 3.01 | 15830 | 29145 |
| Example 6 | F | α-NPD | 4.00 | 3.75 | 14870 | 27043 |
| Example 7 | I | α-NPD | 4.73 | 3.48 | 13930 | 32946 |
| Example 8 | J | α-NPD | 3.21 | 2.97 | 11274 | 30111 |
| Example 9 | K | α-NPD | 4.14 | 2.67 | 12924 | 30535 |
| Example 10 | CuPC | C | 3.51 | 2.66 | 8360 | 29326 |
| Example 11 | I | C | 4.76 | 3.32 | 14434 | 31260 |
| Example 12 | D | C | 4.57 | 2.54 | 10545 | 30355 |
| Example 13 | I | D | 4.25 | 1.77 | 11140 | 32165 |
| Comparative 1 | CuPC | α-NPD | 4.00 | 2.20 | 8019 | 28710 |

As clear from the results in shown in Table 3, the organic electroluminescence element prepared using the carbazole derivatives of the invention has a much more improved luminance half-life period than the conventional organic electroluminescence elements. The organic electroluminescence element of the invention has also an improved efficiency in most cases.

## Claims

1. A carbazole derivative represented by the general formula (I) 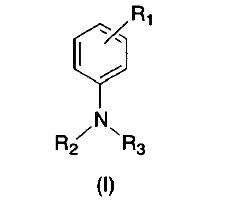 in which R₁ is a hydrogen atom, an alkyl group of 1 to 4 carbon atoms, a cycloalkyl group of 5 to 7 carbon atoms, or an aryl group of 6 to 25 carbon atoms, and R₂ and R₃ are independently an aryl group of 6 to 25 carbon atoms, or any one of first aromatic groups represented by the general formula (II), (III) or (IV) 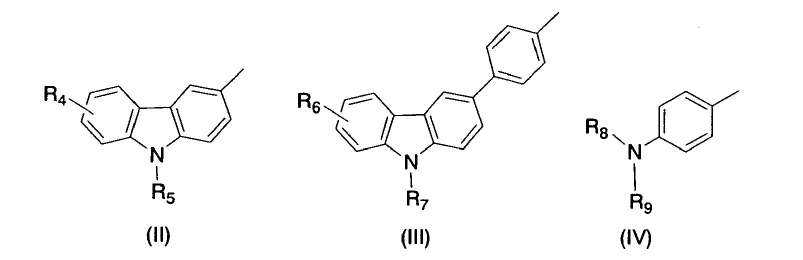 in which R₄ and R₆ are independently a hydrogen atom, an aryl group of 6 to 25 carbon atoms, a diphenylamino group, or a carbazolyl group, and R₅ and R₇ are independently an aryl group of 6 to 25 carbon atoms, a 9-phenyl-3-carbazolyl group, 4-(9-carbazolyl)phenylene group, or 4-diphenylaminophenyl group, and R₈ and R₉ are independently an aryl group of 6 to 25 carbon atoms, or any of second aromatic groups represented by the general formula (V), (VI), or (VII) 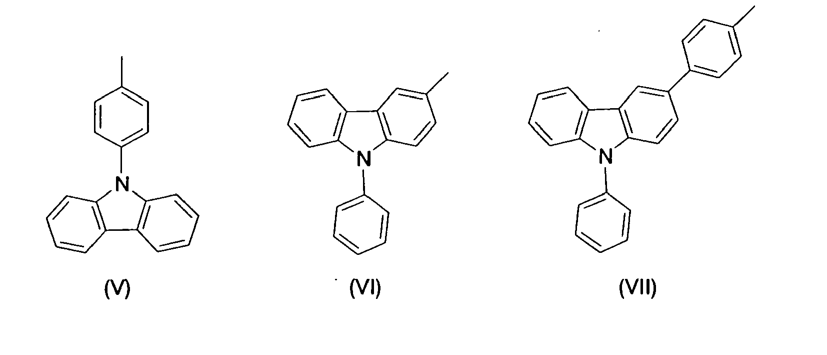 and having at least two carbazole structures in the molecule.

2. An organo-electronic functional material comprising the carbazole derivative as claimed in claim 1.

3. An organic electroluminescence element comprising the organo-electronic functional material as claimed in claim 2.

4. An organic electroluminescence element comprising the organo-electronic functional material as claimed in claim 2 as a hole injecting agent and/or a hole transporting agent.
